# EUROPEAN PATENT SPECIFICATION

(11) **EP 3 967 319 B1**
(45) Date of publication and mention of the grant of the patent: **04.03.2026**
(21) Application number: 21196310.3
(22) Date of filing: 13.09.2021
(51) Int. Cl.: A61K 36/82, A23L 33/105, A61K 8/60, A61K 9/00, A61K 31/7048, A61P 17/00, A61K 8/9789, A61Q 19/00, A61Q 19/08

(54) **COMPOSITION FOR SKIN PROTECTION COMPRISING KAEMPFEROL SACCHARIDES COMPOUNDS**
ZUSAMMENSETZUNG FÜR HAUTSCHUTZ MIT KÄMPFEROL-SACCHARID-VERBINDUNGEN
COMPOSITION POUR LA PROTECTION DE LA PEAU COMPRENANT DES COMPOSÉS DE SACCHARIDES DE KAEMPFEROL

(30) Priority: 15.09.2020 KR 20200118396
(43) Date of publication of application: 16.03.2022
(73) Proprietor: Amorepacific Corporation, Seoul 04386 (KR)
(72) Inventor: KO, Jaeyoung, Gyeonggi-do 17074 (KR); CHO, Si Young, Gyeonggi-do 17074 (KR); KIM, Hyung Su, Gyeonggi-do 17074 (KR); CHO, Sungyeon, Gyeonggi-do 17074 (KR); KIM, Hyoung June, Gyeonggi-do 17074 (KR); BAEK, Heungsoo, Yongin-si Gyeonggi-do 17074 (KR); SON, Euidong, Gyeonggi-do 17074 (KR)
(74) Representative: Germain Maureau

(56) References cited:
- JP-A- 2008 137 998
- JP-A- 2008 201 773
- JP-A- 2009 155 317
- KO JAEYOUNG ET AL: "Kaempferol tri- and tetrasaccharides from Camellia japonica seed cake and their inhibitory activities against matrix metalloproteinase-1 secretion using human dermal fibroblasts", CARBOHYDRATE RESEARCH, PERGAMON, GB, vol. 495, 14 July 2020 (2020-07-14), XP086251016, ISSN: 0008-6215, [retrieved on 20200714], DOI: 10.1016/J.CARRES.2020.108101
- SATO NAOKO ET AL: "Flavonoid glycosides from Japanese Camellia oil cakes and their inhibitory activity against advanced glycation end-products formation", JOURNAL OF FUNCTIONAL FOODS, ELSEVIER BV, NL, vol. 35, 23 May 2017 (2017-05-23), pages 159 - 165, XP085116414, ISSN: 1756-4646, DOI: 10.1016/J.JFF.2017.05.043

## Description

### [Technical Field]

The present disclosure relates to a composition for use in skin protection containing a kaempferol saccharide compound which is safe for human body and has superior skin-protecting effect, wherein the skin protection is improving, preventing or treating atopic skin.

### [Background Art]

Skin is the primary protective barrier of human body. It protects the organs of the body from the change in temperature and humidity and the stimuli of external environments such as UV, pollutants, etc. It also plays an important role in the maintenance of homoeostasis such as temperature control, etc.

However, external factors such as excessive physical and chemical stimuli and stresses, malnutrition, etc. and aging lead to decline in normal skin function and facilitates such phenomena as elasticity loss, cornification, wrinkle formation, etc. In order to prevent these phenomena and maintain healthier skin with elasticity, there have been efforts to maintain the intrinsic function of skin and effectively promote skin regeneration through activation of skin cells by using cosmetic products augmented with physiologically active substances.

However, many of the existing ingredients of cosmetic products have only limited effects or, when they have strong effects, they have many problems such as side effects on skin. Accordingly, interest in ingredients that can promote skin regeneration effectively and regulate skin cornification cycle while being safe for skin is increasing.

Ko Kaeyoung et al., Kaempferol tri- and tetrasaccharides from Camellia japonica seed cake and their inhibitory activities against matrix metalloproteinase-1 (MMP-1) secretion using human dermal fibroblasts, Carbohydrate research, Pergamon, GB, vol. 495, 14 July 2020, discloses four kaempferol tetrasaccharides from *Camellia japonica* seed cake and their MMP-1 inhibitory activities for the development of anti-aging skin cosmetics.

Sato Naoko et al., Flavonoid glycosides from Japanese Camellia oil cakes and their inhibitory activity against advanced glycation end-products formation, Journal of Functional Foods, Elsevier BV, NL, vol. 35, 23 May 2017, pages 159-165, discloses flavonoid glycosides from Japanese Camellia oil cakes and their inhibitory activity against advanced glycation end-products (AGEs) formation.

JP 2008 137998 A discloses a composition useful in food/beverage products for preventing skin aging which comprises water-based component of defatted seeds of *Camellia japonica.*

JP 2008 201773 A discloses a cosmetic external composition for preventing skin aging which comprises water-based component of defatted seeds of *Camellia japonica.*

JP 2009 155317 A discloses a cosmetic external composition for treating skin inflammation which comprises water-based component of defatted seeds of *Camellia japonica.*

### [Disclosure]

### [Technical Problem]

In a non-claimed embodiment, it is disclosed a composition containing a kaempferol saccharide compound as an active ingredient, which has superior skin-protecting effect while being safe for the human body with few side effects.

In a non-claimed embodiment, it is disclosed a composition for skin protection containing a kaempferol saccharide compound extracted from *Camellia japonica* seed cake, which is a waste resource discarded after extraction of *Camellia japonica* seed oil, as an active ingredient.

In a non-claimed embodiment, it is disclosed a composition which improves skin wrinkles or promotes skin regeneration.

In a non-claimed embodiment, it is disclosed a composition which inhibits the expression of matrix metalloprotease-1 (MMP-1).

The present disclosure is directed to providing a composition for use in improving atopic skin.

In a non-claimed embodiment, it is disclosed a composition which regulates skin cornification cycle, regulates skin cornification, facilitates skin cornification turnover, or normalizes skin cornification turnover cycle.

In a non-claimed embodiment, it is disclosed a composition which improves atopic skin or improves, prevents or treats atopic dermatitis by inhibiting the activity of 3-phosphoinositide-dependent kinase 1 (PDK1).

In a non-claimed embodiment, it is disclosed a composition which regulates skin cornification cycle, regulates skin cornification, facilitates skin cornification turnover, or normalizes skin cornification turnover cycle by inhibiting the activity of 3-phosphoinositide-dependent kinase 1 (PDK1).

### [Technical Solution]

The present disclosure provides a composition which contains a kaempferol saccharide compound represented by any of Chemical Formulas 1 to 4, an isomer thereof, a pharmaceutically acceptable salt thereof, a hydrate thereof or a solvate thereof as an active ingredient. 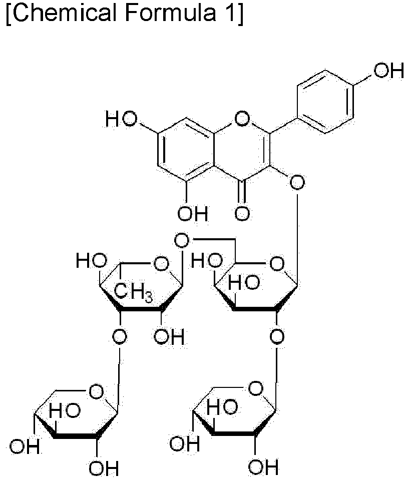 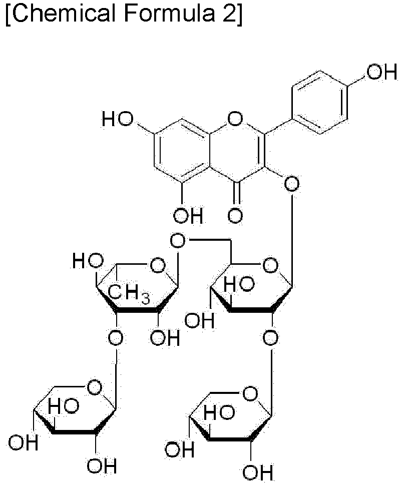 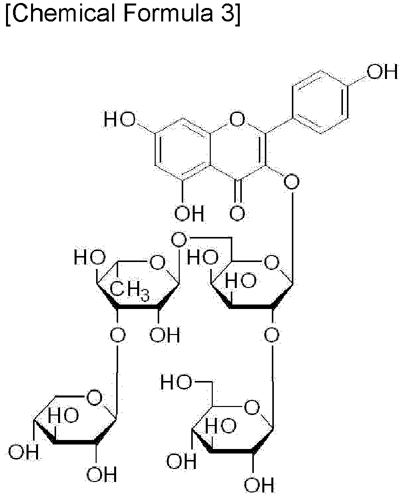 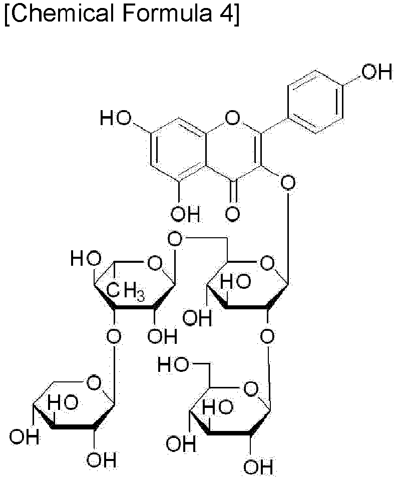 for use in skin protection, wherein the skin protection is improving, preventing or treating atopic skin.

### [Advantageous Effects]

In a non-claimed embodiment, it is disclosed a composition for skin protection disclosed in the present disclosure, which contains a kaempferol saccharide compound, particularly a kaempferol tetrasaccharide compound, as an active ingredient, may improve skin wrinkles and promote skin regeneration by inhibiting the expression of matrix metalloprotease-1 (MMP-1).

In a non-claimed embodiment, it is disclosed a composition for skin protection disclosed in the present disclosure, which contains a kaempferol tetrasaccharide compound as an active ingredient, may improve atopic skin, improve, prevent or treat atopic dermatitis, and regulate skin cornification cycle by inhibiting the activity of 3-phosphoinositide-dependent kinase 1 (PDK1).

### [Brief Description of Drawings]

FIG. 1 shows a result of observing the expression of the senescence marker SA-beta-gal according to an exemplary embodiment of the present disclosure.
FIGS. 2A and 2B show the 3-phosphoinositide-dependent kinase 1 (PDK1) activity-inhibiting effect of a composition according to an exemplary embodiment of the present disclosure.
FIG. 3 shows the matrix metalloprotease-1 (MMP-1) expression-inhibiting effect of a composition according to an exemplary embodiment of the present disclosure.
FIG. 4 shows the effect of a composition according to an exemplary embodiment of the present disclosure on H&E, Ki-67, KRT10, FLG and INV.
FIGS. 5A-5B show the effect of a composition according to an exemplary embodiment of the present disclosure on epidermal thickness and Ki-67.
FIGS. 6A-6C show the effect of a composition according to an exemplary embodiment of the present disclosure on KRT10, FLG and INV.
FIG. 7 shows the information of the kaempferol saccharide compounds represented by Chemical Formula 1-4.
FIGS. 8A-8B schematically illustrate an experiment of testing the recovery of regeneration ability upon inhibition of PDK1.
FIGS. 9A-9B show the functional recovery of skin cells upon inhibition of PDK1.
FIGS. 10A-10B show the effect of PDK1 on skin regeneration.
FIGS. 11A-11B show that the inhibition of PDK1 inhibits SASP in a skin equivalent.

### [Best Mode]

In the present disclosure, when a certain portion is described to "comprise" a certain element, it means that another element may be further included unless particularly specified otherwise.

Hereinafter, the present disclosure is described in detail.

In an exemplary embodiment, the present disclosure provides a composition for skin protection containing a kaempferol saccharide compound represented by any of Chemical Formulas 1 to 4, an isomer thereof, a pharmaceutically acceptable salt thereof, a hydrate thereof or a solvate thereof as an active ingredient. 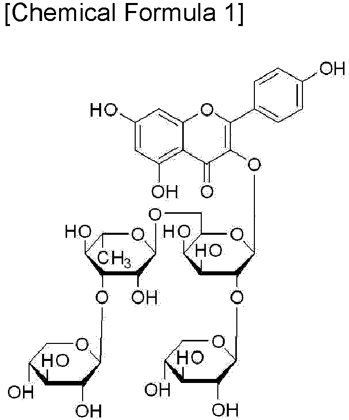 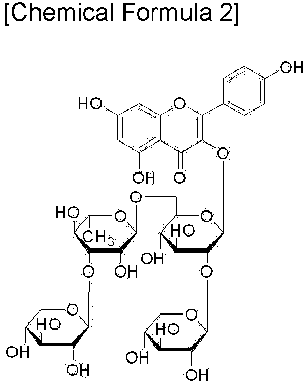 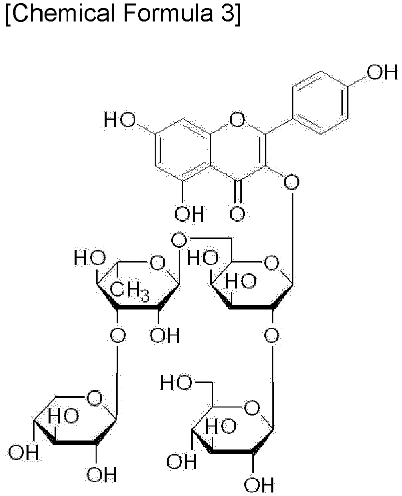 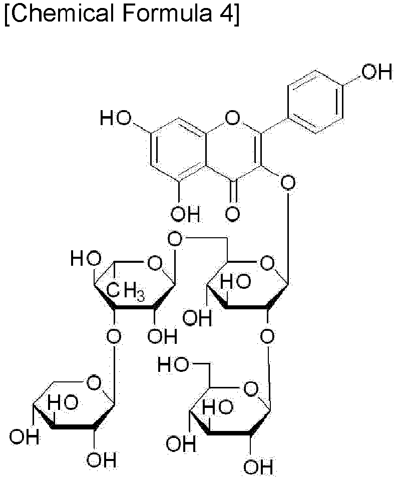 for use in skin protection, wherein the skin protection is improving, preventing or treating atopic skin.

**In** the present disclosure, the "isomer" particularly includes not only optical isomers (e.g., essentially pure enantiomers, essentially pure diastereomers or mixtures thereof) but also conformational isomers (i.e., isomers differing only in the angle of one or more chemical bond), position isomers (particularly tautomers) or geometric isomers (e.g., cis-trans isomers).

**In** the present disclosure, "essentially pure" means that, when use in connection with, for example, enantiomers or diastereomers, a specific compound is present at about 90% (w/w) or more, specifically about 95% or more, more specifically about 97% or more or about 98% or more, even more specifically about 99% or more, yet more specifically about 99.5% or more.

**In** the present disclosure, "pharmaceutically acceptable" means being recognized as being approvable or approved by the government or a regulatory agency equivalent thereto for use in animals, more particularly in human, by avoiding significant toxic effects when used in conventional medicinal dosages or as being listed or described in the Pharmacopeia or other general pharmacopeias.

In the present disclosure, the "pharmaceutically acceptable salt" means a salt according to an aspect of the present disclosure which is pharmaceutically acceptable and has the desired pharmacological activity of the parent compound. The salt may include: (1) an acid addition salt formed from an inorganic acid such as hydrochloric acid, hydrobromic acid, sulfuric acid, nitric acid, phosphoric acid, etc., or an organic acid such as acetic acid, priopionic acid, hexanoic acid, cyclopentylpropionic acid, glycolic acid, pyruvic acid, lactic acid, malonic acid, succinic acid, malic acid, maleic acid, fumaric acid, tartaric acid, citric acid, benzoic acid, 3-(4-hydroxybenzoyl)benzoic acid, cinnamic acid, mandelic acid, methanesulfonic acid, ethanesulfonic acid, ethane-1,2-disulfonic acid, 2-hydroxyethanesulfonic acid, benzenesulfonic acid, 4-chlorobenzenesulfonic acid, 2-naphthalenesulfonic acid, 4-toluenesulfonic acid, camphorsulfonic acid, 4-methylbicyclo[2,2,2]-oct-2-ene-1-carboxylic acid, glucoheptonic acid, 3-phenylpropionic acid, trimethylacetic acid, tert-butylacetic acid, lauryl sulfuric acid, gluconic acid, glutamic acid, hydroxynaphthoic acid, salicylic acid, stearic acid or muconic acid; or (2) a salt formed when an acidic proton present in the parent compound is substituted.

In the present disclosure, the "hydrate" means a compound to which water is bound and is used in a broad concept, including an inclusion compound lacking chemical bonding between water and the compound.

In the present disclosure, the "solvate" means a higher-order compound formed from a molecule or ion of a solute and a molecule or ion of a solvent.

The IUPAC name of the kaempferol saccharide compound represented by Chemical Formula 1 is kaempferol-3-O-β-D-xylopyranosyl-(1→3)-O-α-L-rhamnopyranosyl-(1→6)-O-β-D-xylopyranosyl-(1→2)-O-β-D-galactopyranoside. It can exist as pale yellow powder at room temperature.

The information of the kaempferol saccharide compound represented by Chemical Formula 1 is as follows.

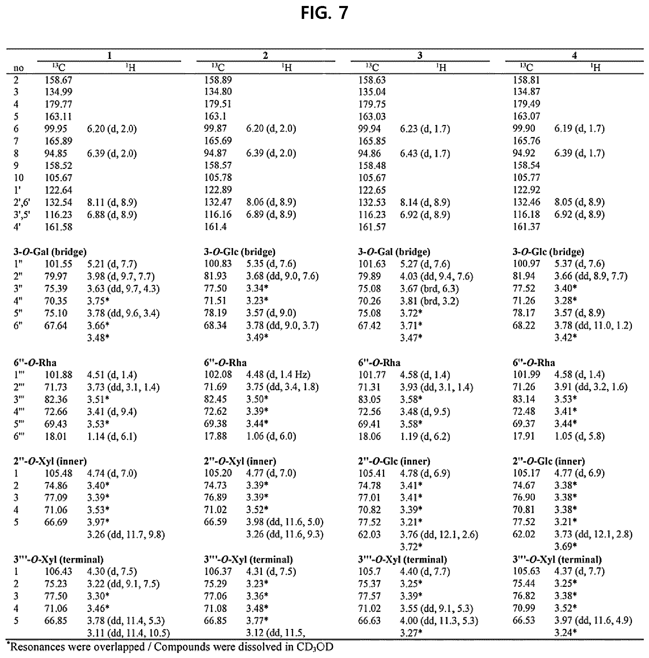

Pale yellow powder; UV (HPLC-PDA) λₘₐₓ 266, 347 nm; [α]²²_{D} -57.4 (c 0.10); ESI-Q-TOF-MS: m/z 859.2503 [M+H]⁺ (calcd. for C₃₇H₄₇O₂₃⁺, 859.2508), 881.2329 [M+Na]⁺ (calcd. for C₃₇H₄₆O₂₃Na⁺, 859.2327); ¹H (CD₃OD, 500 MHz) and ¹³C NMR (CD₃OD, 125 MHz) (see FIG. 7).

The IUPAC name of the kaempferol saccharide compound represented by Chemical Formula 2 is kaempferol-3-O-β-D-xylopyranosyl-(1→3)-O-α-L-rhamnopyranosyl-(1→6)-O-β-D-xylopyranosyl-(1→2)-O-β-D-glucopyranoside. It can exist as pale yellow powder at room temperature.

The information of the kaempferol saccharide compound represented by Chemical Formula 2 is as follows.

Pale yellow powder; UV (HPLC-PDA) λₘₐₓ 266, 347 nm; [α]²²_{D} -63.7 (c 0.10); ESI-Q-TOF-MS: m/z 859.2505 [M+H]⁺ (calcd. for C₃₇H₄₇O₂₃⁺, 859.2508), 881.2325 [M+Na]⁺ (calcd. for C₃₇H₄₆O₂₃Na⁺, 859.2327); ¹H (CD₃OD, 500 MHz) and ¹³C NMR (CD₃OD, 125 MHz) (see FIG. 7).

The IUPAC name of the kaempferol saccharide compound represented by Chemical Formula 3 is kaempferol-3-O-β-D-xylopyranosyl-(1→3)-O-α-L-rhamnopyranosyl-(1→6)-O-β-D-glucopyranosyl-(1→2)-O-β-D-galactopyranoside. It can exist as pale yellow powder at room temperature.

The information of the kaempferol saccharide compound represented by Chemical Formula 3 is as follows.

Pale yellow powder; UV (HPLC-PDA) λₘₐₓ 266, 347 nm; [α]²²_{D} -72.5 (c 0.2); ESI-Q-TOF-MS: m/z 889.2616 [M+H]⁺ (calcd. for C₃₈H₄₉O₂₃⁺, 889.2613) and 911.2432 [M+Na]⁺ (calcd. for C₃₈H₄₈O₂₄Na⁺, 859.2327); ¹H (CD₃OD, 500 MHz) and ¹³C NMR (CD₃OD, 125 MHz) (see FIG. 7).

The IUPAC name of the kaempferol saccharide compound represented by Chemical Formula 4 is kaempferol-3-O-β-D-xylopyranosyl-(1→3)-O-α-L-rhamnopyranosyl-(1→6)-O-β-D-glucopyranosyl-(1→2)-O-β-D-glucopyranoside. It can exist as pale yellow powder at room temperature.

The information of the kaempferol saccharide compound represented by Chemical Formula 4 is as follows.

Pale yellow powder; UV (HPLC-PDA) λₘₐₓ 266, 347 nm; [α]²²_{D} -45.1 (c 0.10); ESI-Q-TOF-MS: m/z 889.2613 [M+H]⁺ (calcd. for C₃₈H₄₉O₂₃⁺, 889.2613) and 911.2423 [M+Na]⁺ (calcd. for C₃₈H₄₈O₂₄Na⁺, 859.2327); ¹H (CD₃OD, 500 MHz) and ¹³C NMR (CD₃OD, 125 MHz) (see FIG. 7).

The composition according to an aspect of the present disclosure, which contains a kaempferol tetrasaccharide compound extracted from *Camellia japonica* seed cake discarded after extraction of oil from *Camellia japonica* seed as an active ingredient, can exhibit remarkably superior effect of improving, preventing or treating atopic dermatitis as compared to a composition containing the existing kaempferol saccharide compound as an active ingredient.

*Camellia japonica* seed cake extract contains terpene saccharides, flavonoid saccharides, sugars and proteins. In particular, the flavonoid saccharides of the *Camellia japonica* seed cake include kaempferol tetrasaccharides (Chemical Formulas 1, 2, 3 and 4) and trisaccharides (Chemical Formulas 5, 6, 7 and 8). The inventors of the present disclosure have identified that, among the kaempferol saccharides derived from the *Camellia japonica* seed cake, the four tetrasaccharides (Chemical Formulas 1, 2, 3 and 4) have excellent skin-protecting effect.

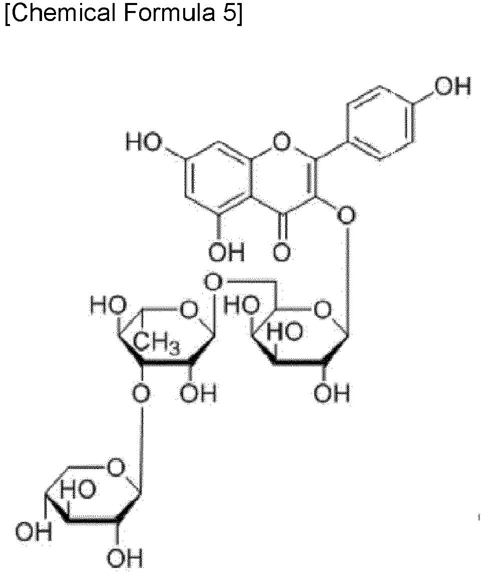

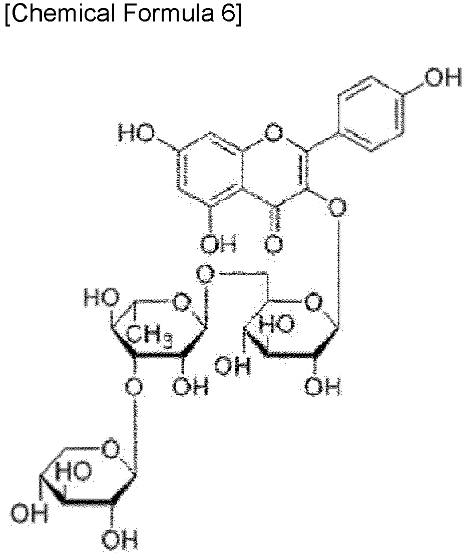

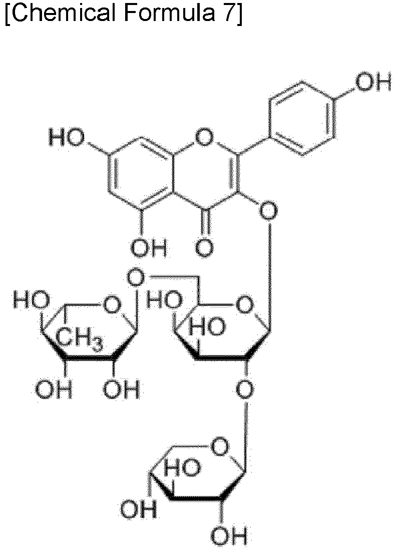

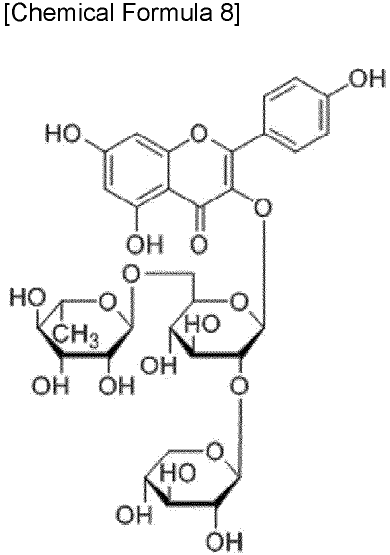

**In** an exemplary embodiment, the kaempferol saccharide compound may be one extracted from *Camellia japonica* seed cake.

Specifically, the kaempferol saccharide compound may be obtained by obtaining *Camellia japonica* seed cake which is obtained after producing oil from *Camellia japonica* seed through compression and then extracting a kaempferol saccharide compound from the *Camellia japonica* seed cake using 70% ethanol.

In an exemplary embodiment, the concentration of the active ingredient may be 0.01-100 µM, for example, 0.01 µM or higher, 0.1 µM or higher, 0.5 µM or higher, 1 µM or higher, 1.5 µM or higher, 2 µM or higher or 2.5 µM or higher, and 100 µM or lower, 50 µM or lower, 10 µM or lower, 8 µM or lower, 6 µM or lower or 4 µM or lower, based on the total weight of the composition.

When the concentration is lower than 0.01 µM, the skin-protecting effect such as promotion of skin regeneration, improvement of atopic dermatitis, regulation of skin cornification cycle, etc. may be insignificant. And, when it exceeds 100 µM, cytotoxicity may occur.

In a non-claimed embodiment, the composition may be for improving skin wrinkles.

In a non-claimed embodiment, the composition may be for promoting skin regeneration.

In a non-claimed embodiment, the composition may be for inhibiting the expression of matrix metalloprotease-1 (MMP-1).

In a non-claimed embodiment, the composition may be for improving skin wrinkles by inhibiting the expression of matrix metalloprotease-1 (MMP-1).

In a non-claimed embodiment, the composition may be for promoting skin regeneration by inhibiting the expression of matrix metalloprotease-1 (MMP-1).

The composition is for use in improving atopic skin.

In a non-claimed embodiment, the composition may be for regulating skin cornification cycle.

The skin cornification cycle, also called the turnover cycle of epidermal cells, means the cycle of formation of degenerated corneocytes on skin surface from keratinocytes at the bottom of the epidermis.

The turnover cycle of skin cells may be determined by two factors; either by measuring colony formation efficiency while maintaining the growth potential of epidermal cells or by measuring the differentiation level of the epidermal cells. In an aspect of the present disclosure, the composition, which contains the kaempferol tetrasaccharide compound as an active ingredient, is for use in reducing the skin cornification cycle, or the turnover cycle of epidermal cells, by facilitating colony formation and differentiation of epidermal cells.

In a non-claimed embodiment, the composition may be for regulating skin cornification, for facilitating skin cornification turnover, or for normalizing skin cornification turnover cycle.

In a non-claimed embodiment, the composition may inhibit the activity of 3-phosphoinositide-dependent kinase 1 (PDK1).

When the activity of PDK1 is inhibited using a composition containing the kaempferol saccharide compound represented by any of Chemical Formulas 1 to 4, the cells growth of which has been inhibited due to aging may grow again, and damaged cells may be recovered.

In other words, the inhibition of PDK1 in aged human dermal fibroblasts may recover or promote skin regeneration ability by suppressing aging characteristics and inhibiting NF-kB and mTOR signal transduction, thereby recovering cell cycle.

In a non-claimed embodiment, the composition may be for improving skin wrinkles by inhibiting the activity of 3-phosphoinositide-dependent kinase 1 (PDK1).

In a non-claimed embodiment, the composition may be for promoting skin regeneration by inhibiting the activity of 3-phosphoinositide-dependent kinase 1 (PDK1).

In a non-claimed embodiment, the composition may be for improving atopic skin, or improving, preventing or treating atopic dermatitis by inhibiting the activity of 3-phosphoinositide-dependent kinase 1 (PDK1).

In a non-claimed embodiment, the composition may be for regulating skin cornification cycle by inhibiting the activity of 3-phosphoinositide-dependent kinase 1 (PDK1).

In a non-claimed embodiment, the composition may be for regulating skin cornification by inhibiting the activity of 3-phosphoinositide-dependent kinase 1 (PDK1).

In a non-claimed embodiment, the composition may be for facilitating skin cornification turnover by inhibiting the activity of 3-phosphoinositide-dependent kinase 1 (PDK1).

In a non-claimed embodiment, the composition may be for normalizing skin cornification turnover cycle by inhibiting the activity of 3-phosphoinositide-dependent kinase 1 (PDK1).

The composition is for use in improving skin barrier. It inhibits the activity of 3-phosphoinositide-dependent kinase 1 (PDK1).

In an exemplary embodiment, the composition may be a cosmetic composition.

The cosmetic composition according to an exemplary embodiment of the present disclosure may contain a cosmetologically or dermatologically acceptable medium or base. It may be prepared into any topically applicable formulation, for example, in the form of a solution, a gel, a solid, an anhydrous paste, an oil-in-water emulsion, a suspension, a microemulsion, a microcapsule, a microgranule, an ionic (liposomal) or nonionic vesicular dispersion, a cream, a lotion, a powder, an ointment, a spray or a concealer stick. These compositions may be prepared according to common methods in the art. The composition according to the present disclosure may also be used in the form of a foam or an aerosol composition further containing a compressed propellant.

The cosmetic composition according to an exemplary embodiment of the present disclosure may be formulated into cosmetics such as a softening lotion, an astringent lotion, a nourishing lotion, a nourishing cream, a massage cream, an essence, an eye cream, an eye essence, a cleansing cream, a cleansing foam, a cleansing water, a pack, a powder, a body lotion, a body cream, a body oil, a body essence, etc.

When the formulation according to an exemplary embodiment of the present disclosure is a paste, a cream or a gel, animal fiber, plant fiber, wax, paraffin, starch, tragacanth, a cellulose derivative, polyethylene glycol, silicon, bentonite, silica, talc, zinc oxide, etc. may be used as a carrier ingredient.

When the formulation according to an exemplary embodiment of the present disclosure is a powder or a spray, lactose, talc, silica, aluminum hydroxide, calcium silicate or polyamide powder may be used as a carrier ingredient. Particularly, a spray may additionally contain a propellant such as chlorofluorohydrocarbon, propane/butane or dimethyl ether.

When the formulation according to an exemplary embodiment of the present disclosure is a solution or an emulsion, a solvent, solubilizer or an emulsifier may be used as a carrier ingredient. Examples include water, ethanol, isopropanol, ethyl carbonate, ethyl acetate, benzyl alcohol, benzyl benzoate, propylene glycol, 1,3-butylene glycol, aliphatic glycerol ester, polyethylene glycol or a fatty acid ester of sorbitan.

When the formulation according to an exemplary embodiment of the present disclosure is a suspension, a liquid diluent such as water, ethanol or propylene glycol, a suspending agent such as ethoxylated isostearyl alcohol, polyoxyethylene sorbitol ester and polyoxyethylene sorbitan ester, microcrystalline cellulose, aluminum metahydroxide, bentonite, agar, tragacanth, etc. may be used as a carrier ingredient.

When the formulation according to an exemplary embodiment of the present disclosure is a surfactant-containing cleanser, aliphatic alcohol sulfate, aliphatic alcohol ether sulfate, sulfosuccinic monoester, isethionate, an imidazolinium derivative, methyl taurate, sarcosinate, a fatty acid amide ether sulfate, an amidoalkyl betaine, an aliphatic alcohol, a fatty acid glyceride, a fatty acid diethanolamide, vegetable oil, a lanolin derivative, an ethoxylated glycerol fatty acid ester, etc. may be used as a carrier ingredient.

The cosmetic composition according to an exemplary embodiment of the present disclosure may further contain, in addition to the active ingredient, a functional additive and ingredients contained in general cosmetic compositions. The functional additive may be an ingredient selected from a group consisting of a water-soluble vitamin, an oil-soluble vitamin, a polypeptide, a polysaccharide, a sphingolipid and a seaweed extract.

In addition, the cosmetic composition according to an exemplary embodiment of the present disclosure may further contain ingredients contained in general cosmetic compositions together with the functional additive, if necessary. In addition, an ingredient such as an oil, a fat, a humectant, an emollient, a surfactant, an organic or inorganic pigment, an organic powder, a UV absorbent, an antiseptic, a sterilizer, an antioxidant, a plant extract, a pH control agent, an alcohol, a colorant, a flavorant, a blood circulation stimulant, a cooling agent, an antiperspirant, purified water, etc. may be further contained.

In addition, the composition according to an exemplary embodiment of the present disclosure may be a composition for external application to skin. The composition for external application to skin collectively refers to any form that can be applied externally to skin, and various formulations of cosmetics and pharmaceuticals may be included therein.

In an exemplary embodiment, the composition may be a pharmaceutical composition for improving, preventing or treating atopic dermatitis.

The pharmaceutical composition according to an exemplary embodiment of the present disclosure may further contain a pharmaceutical adjuvant such as an antiseptic, a stabilizer, a wetting agent, an emulsification promoter, a salt and/or a buffer for controlling osmotic pressure, etc. and other therapeutically useful substances, and may be formulated into various forms for oral administration or parenteral administration.

The formulation for oral administration may be, for example, a tablet, a pill, a hard or soft capsule, a liquid, a suspension, an emulsion, a syrup, a powder, a dust, a fine granule, a granule, a pellet, etc., and these formulations may contain, in addition to the active ingredient, a surfactant, a diluent (e.g., lactose, dextrose, sucrose, mannitol, sorbitol, cellulose or glycine) or a lubricant (e.g., silica, talc, stearic acid and its magnesium or calcium salt or polyethylene glycol). The tablet may further contain a binder such as magnesium aluminum silicate, starch paste, gelatin, tragacanth, methylcellulose, sodium carboxymethylcellulose and polyvinylpyrrolidone and may contain, in some cases, a pharmaceutical additive such as a disintegrant, e.g., starch, agar, alginic acid or its sodium salt, an absorbent, a colorant, a flavorant, a sweetener, etc. The tablet may be prepared by a common mixing, granulation or coating method. In addition, the formulation for parenteral administration may be a formulation for transdermal administration, e.g., an injection, a medicinal drop, an ointment, a lotion, a gel, a cream, a spray, a suspension, an emulsion, a suppository, a patch, etc.,

The pharmaceutical composition according to an exemplary embodiment of the present disclosure may be administered parenterally, e.g., rectally, topically, transdermally, subcutaneously, etc.

Determination of the administration dosage of the active ingredient is within the level of those skilled in the art. A daily administration dosage of the composition, although it varies depending on various factors such as the severity of a disease to be treated, onset of the disease, age, health condition, complications, etc., may be usually 0.01-100 mg/kg/day, e.g., 0.01 mg/kg/day or more, 0.1 mg/kg/day or more, 0.5 mg/kg/day or more, 1 mg/kg/day or more, 1.3 mg/kg/day or more, 1.5 mg/kg/day or more, 1.7 mg/kg/day or more, 2 mg/kg/day or more or 2.2 mg/kg/day or more, and 100 mg/kg/day or less, 50 mg/kg/day or less, 10 mg/kg/day or less, 7 mg/kg/day or less, 5 mg/kg/day or less, 3 mg/kg/day or less or 2.5 mg/kg/day or less, for an adult. Specifically, a daily administration dosage of 1-5 mg/kg may be administered once to three times a day.

In an exemplary embodiment, the composition may be a food composition.

The food composition according to an exemplary embodiment of the present disclosure may be a formulation in liquid or solid state, and may be a formulation in the form of a tablet, a capsule, a soft capsule, a pill, a granule, a drink, a diet bar, a chocolate, a caramel or confectionery, The food composition of the present disclosure may adequately contain an excipient, a saccharide, a flavorant, a colorant, an oil or fat, a protein, etc. in addition to the active ingredient, if necessary.

In a non-claimed embodiment, it is disclosed a method for protecting skin, which includes administering an effective amount of a composition containing a kaempferol saccharide compound represented by any of Chemical Formulas 1 to 4, an isomer thereof, a pharmaceutically acceptable salt thereof, a hydrate thereof or a solvate thereof as active ingredient to a subject in need thereof.

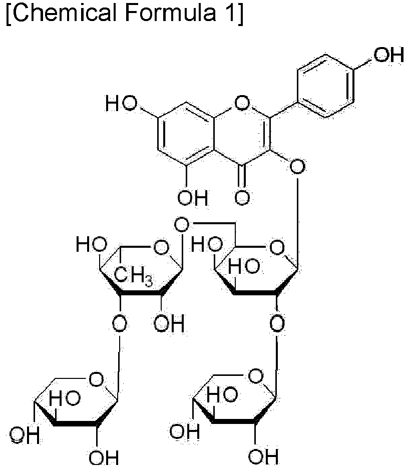

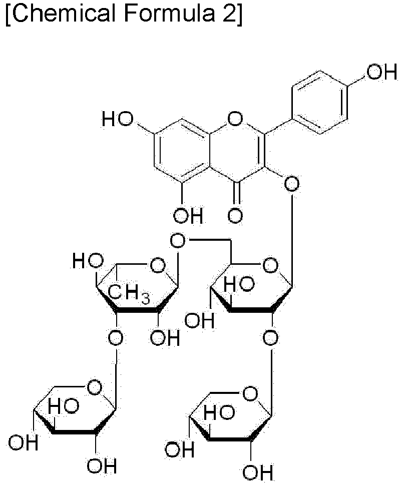

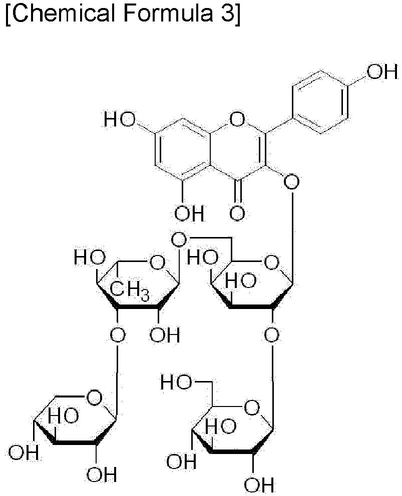

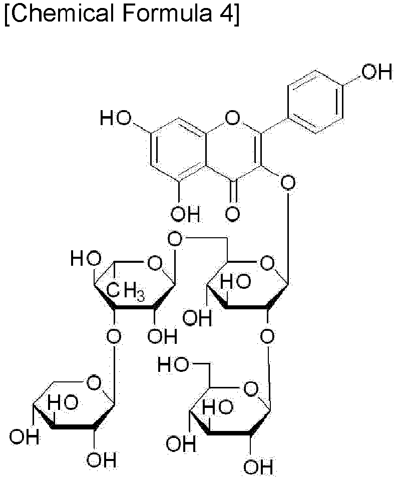

In a non-claimed embodiment, it is disclosed a use of a kaempferol saccharide compound represented by any of Chemical Formulas 1 to 4, an isomer thereof, a pharmaceutically acceptable salt thereof, a hydrate thereof or a solvate thereof for preparation of a composition for skin protection.

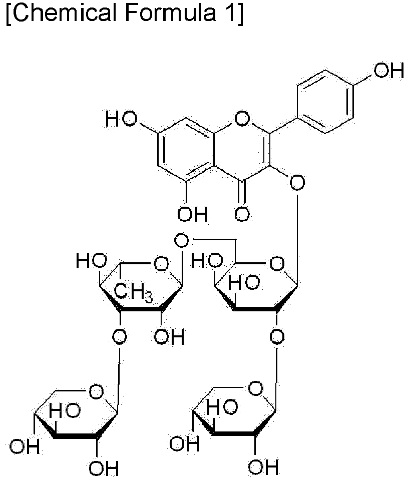

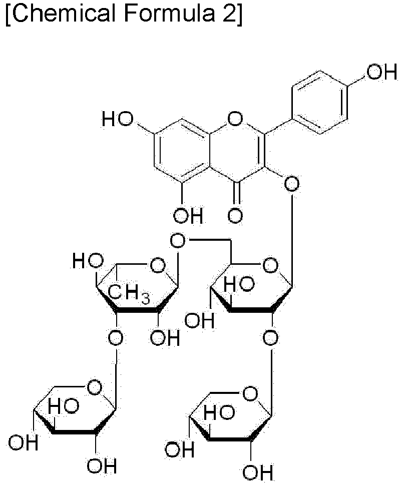

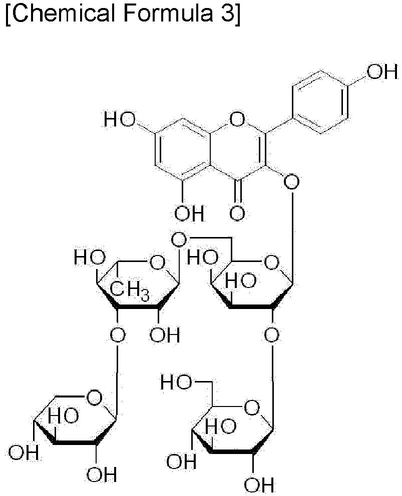

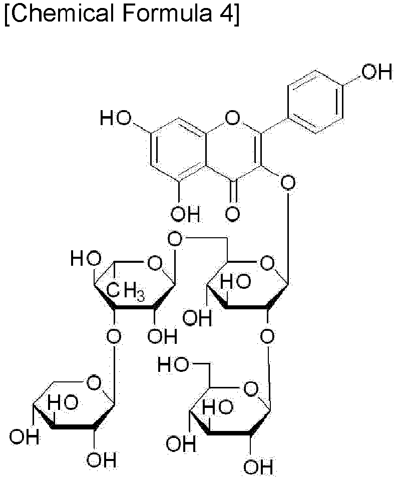

The present disclosure provides a non-therapeutic use of a kaempferol saccharide compound represented by any of Chemical Formulas 1 to 4, an isomer thereof, a pharmaceutically acceptable salt thereof, a hydrate thereof or a solvate thereof for use in protecting skin as claimed in claim 6.

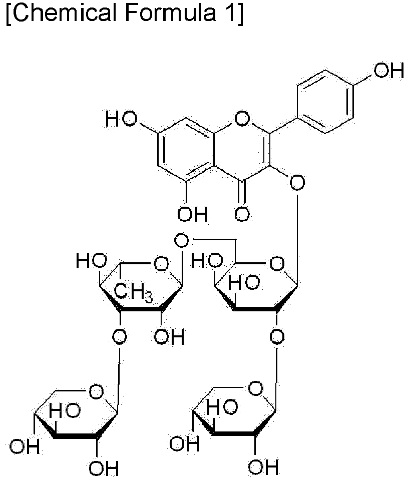

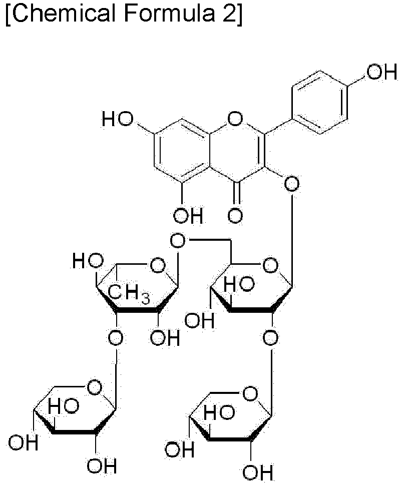

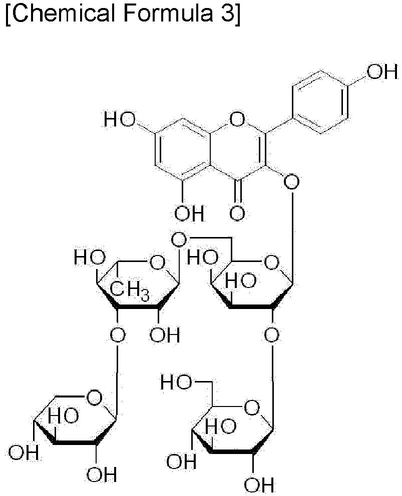

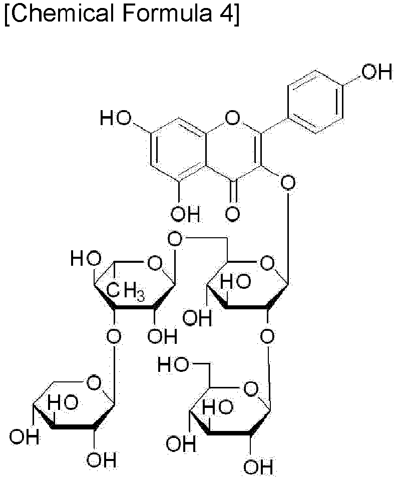

In another exemplary embodiment, the present disclosure provides a kaempferol saccharide compound represented by any of Chemical Formulas 1 to 4, an isomer thereof, a pharmaceutically acceptable salt thereof, a hydrate thereof or a solvate thereof for use in improvement, prevention or treatment of atopic dermatitis.

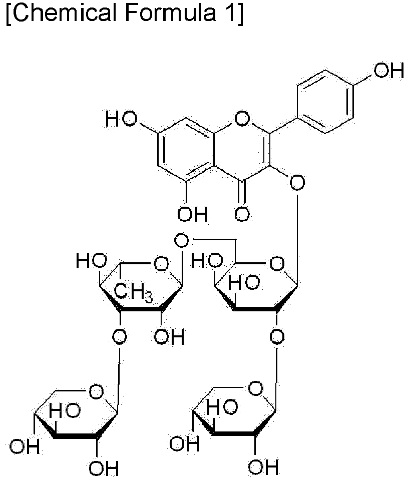

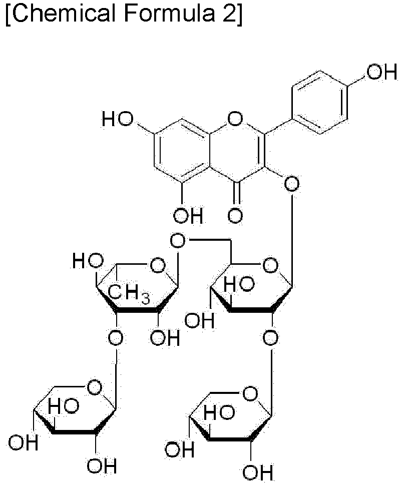

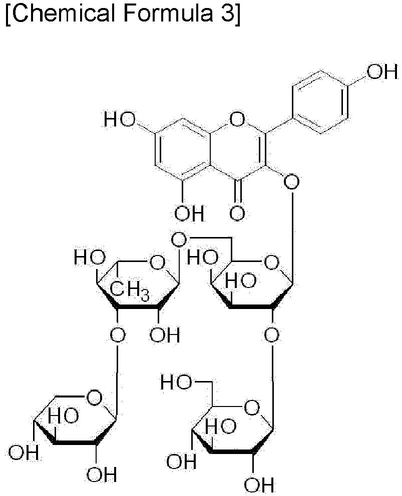

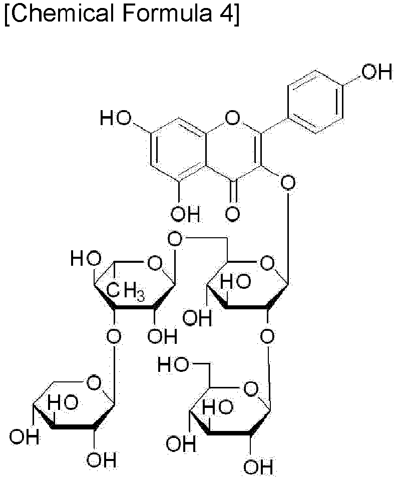

Hereinafter, the present disclosure is described more specifically through examples.

### Example

*Camellia japonica* seed cake was obtained after producing oil from *Camellia japonica* seed harvested in Jeju Dongbaek Town through compression. An extract was prepared from the *Camellia japonica* seed cake using 70% ethanol and it was identified that the *Camellia japonica* seed cake extract contain terpene saccharides, flavonoid saccharides, sugars and proteins. In particular, it was confirmed that the flavonoid saccharides of the *Camellia japonica* seed cake consist of kaempferol tetrasaccharides (Chemical Formulas 1-4) and trisaccharides (Chemical Formulas 5-8) and that, among the kaempferol saccharides of the *Camellia japonica* seed cake, four tetrasaccharides (Chemical Formulas 1-4) have excellent skin antiaging and regeneration effects.

### 1) Purification of kaempferol saccharide fractions of Camellia japonica seed cake

After adding 8 kg of 70% ethanol to 1 kg of *Camellia japonica* seed cake obtained after extracting oil by compression at low temperature, extraction was conducted at 60 °C for 8 hours using a heating extractor. After filtering with a filter press, the obtained extract was concentrated under reduced pressure at 50-60 °C with a rotary vacuum evaporator until the solid content reached about 50%. 200 g of the obtained concentrate was diluted with purified water such that the solid content became 10%.

After loading the solution in a glass tube column packed with 2 L of an ionexchange resin (HP-20), impurities and sugars were removed by washing with 10 L of purified water. Then, after flowing 10 L of 30% ethanol, the eluate was concentrated under reduced pressure and freeze-dried to obtain about 20 g of *Camellia japonica* kaempferol saccharide fraction powder.

### 2) Purification of Camellia japonica seed cake kaempferol tetrasaccharides and trisaccharides

The ingredients of the *Camellia japonica* kaempferol saccharide fraction were separated by fractional HPLC because they have little difference in elution times. A mixture solvent of water, acetonitrile and methanol was used as an eluent. Kaempferol saccharide compounds of Chemical Formulas 1-4 (tetrasaccharides) and 5-8 (trisaccharides) were separated using the solvent with a concentration gradient.

### Test Examples

### Statistics

In all the following experiments, the statistical significance between samples was analyzed by Student's t-test. A p-value of 0.05 or smaller was considered statistically significant. (*p < 0.05, **p < 0.01, ***p < 0.001)

### Test Example 1: Investigation of expression level of senescence marker SA-beta gal

### *Cells and culturing

### (1) Culturing of senescent skin cells

Normal human dermal fibroblasts (NHDF; Lonza, Switzerland) were cultured in Dulbecco's modified Eagle's medium (DMEM, GIBCO 1210-0038) containing 10% fetal bovine serum, in a 5% CO₂ incubator at 37 °C. Cells at passages 6-9 were denoted as young cells, and they were subcultured by 1:5 subculturing until passage 37 to obtain senescent dermal fibroblasts.

### (2) Analysis of expression activity of senescence marker SA-beta gal

The senescent skin cells of (1) were treated with DMSO, the compounds repressed by Chemical Formula 1, 2, 3, 4, 5, 6, 7 and 8, kaempferol 3-O-β-rutinoside (Chemical Formula 9) or kaempferol 7-O-β-D-glucopyranoside (Chemical Formula 10) at 3 µM for 7 days.

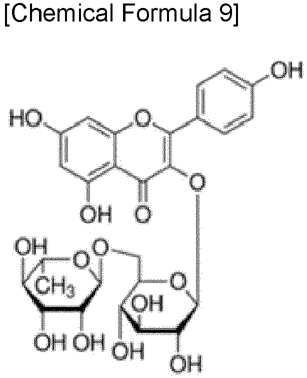

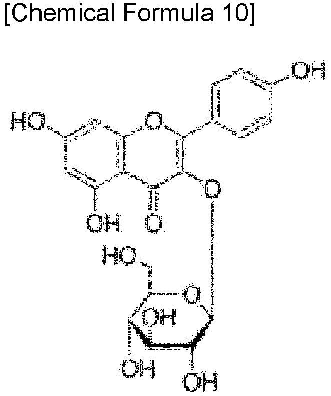

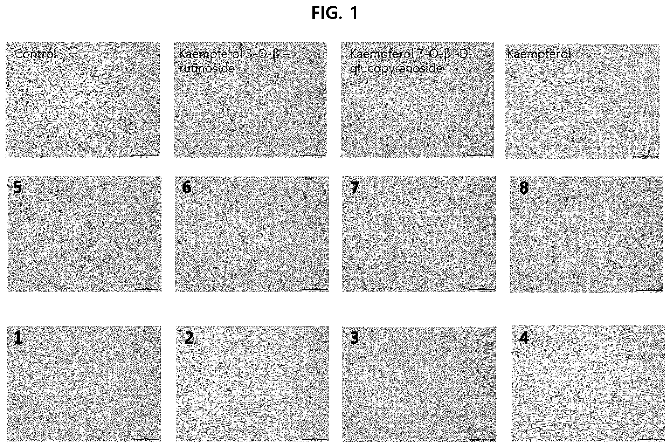

On day 8, the cells were washed with PBS and stained with a fixative and a stain provided together with a cellular senescence assay kit (Cell Biolabs, Inc., San Diego, CA, USA) at 37 °C for 16 hours. On the next day, after washing with PBS and applying a 20% glycerol solution, the number of cells stained green was counted and the degree of expression of the senescence marker SA-beta-Gal was evaluated. The result is shown in Table 1 and FIG. 1.

**[Table 1]**

| Control | Kaempferol | Kaempferol 3-O-β-rutinoside | Kaempferol 7-O-β-D-glucopyranoside | 5 | 6 | 7 | 8 | 1 | 2 | 3 | 4 |
|---|---|---|---|---|---|---|---|---|---|---|---|
| 78± 5.2 | 80 ± 12.1 | 81 ± 11.3 | 79± 9.9 | 79 ± 11.2 | 81 ± 9.7 | 69 ± 21.1 | 75 ± 10.6 | 38 ± 10.4 | 40 ± 10.4 | 42 ± 9.7 | 37 ± 9.1 |

### *Cell viability assay

1. Human dermal fibroblasts (HDF) were seeded onto a 96-well plate at a concentration of 1x10⁴ cells/well and cultured for 48 hours under a cell culturing condition.
2. The cells were treated with the test substances diluted in FBS-free DMEM at different concentrations and then cultured for 48 hours.
3. After washing with Dulbecco's phosphate-buffered saline (DPBS) and replacing the medium with a medium containing 10% EZ-Cytox solution, the cells were cultured for 1 hour under a cell culturing condition and absorbance was measured at 450 nm using an ELISA reader (Tecan Infinite M200 Pro).

Referring to Table 1, it can be seen that the compounds of Chemical Formulas 1-4 of the present disclosure inhibited the expression of the senescence marker SA-beta by about 50% when compared with the control or comparative compounds (Chemical Formulas 5-8).

Also, referring to FIG. 1, it can be seen that the compounds of Chemical Formulas 1-4 of the present disclosure resulted in remarkable decrease of the senescence marker SA-beta gal in the cultured cells.

### Test Example 2: PDK1 assay

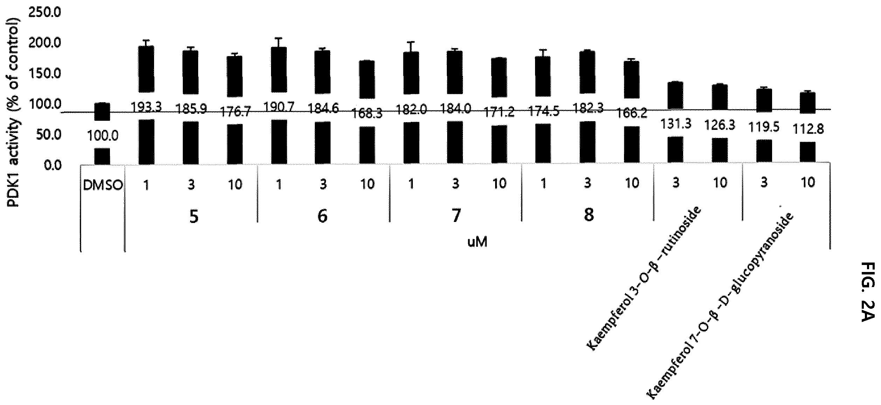

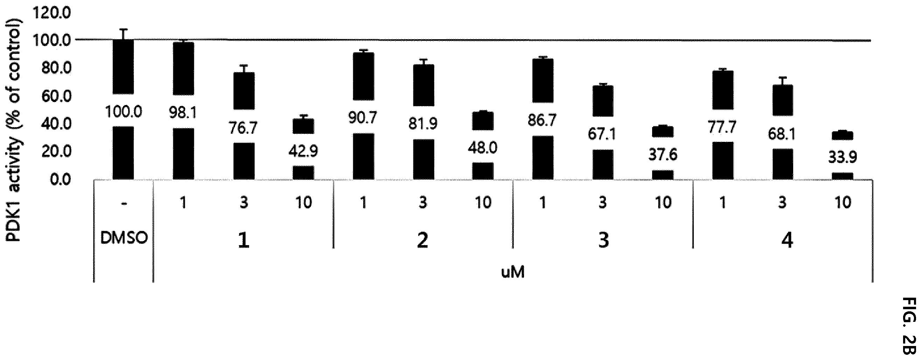

The activity of 3-phosphoinositide-dependent kinase 1 (PDK1) was measured using a recombinant purified PDK1 enzyme and an ADP-Glo + PDK1 kinase enzyme system (Promega, Madison, WI, USA). After adding a mixture of a substrate, ATP and a buffer, the test substance (1, 3, 10 µM) was added to each well. Immediately thereafter, PDK1 was added and reaction was conducted for 60 minutes. After the reaction, ADP-glo was added and incubation was conducted at room temperature for 40 minutes. Then, after adding a kinase detection buffer and conducting reaction for 30 minutes, measurement was made using a luminescence reader (Wallac Victor2, PerkinElmer). The result is shown in FIGS. 2A and 2B.

Referring to FIGS. 2A and 2B, it can be seen that the compounds of Chemical Formulas 1-4 of the present disclosure (FIG. 2B) have superior PDK activity-inhibiting effect as compared to the comparative compounds of Chemical Formulas 5-8 (FIG. 2A). Specifically, referring to FIG. 2A, it can be seen that the compounds of Chemical Formulas 5-8 did not inhibit PDK1 activity but increased it on the contrary as compared to the DMSO control group. In contrast, referring to FIG. 2B, it can be seen that the compounds of Chemical Formulas 1-4 of the present disclosure appreciably inhibited PDK1 activity as compared to the DMSO control group. In addition, it can be seen that, among the kaempferol tetrasaccharide compounds (Chemical Formulas 1-4), the higher effect is achieved at the same concentration as the number of OH group is larger.

### Test Example 3: MMP-1 ELISA assay

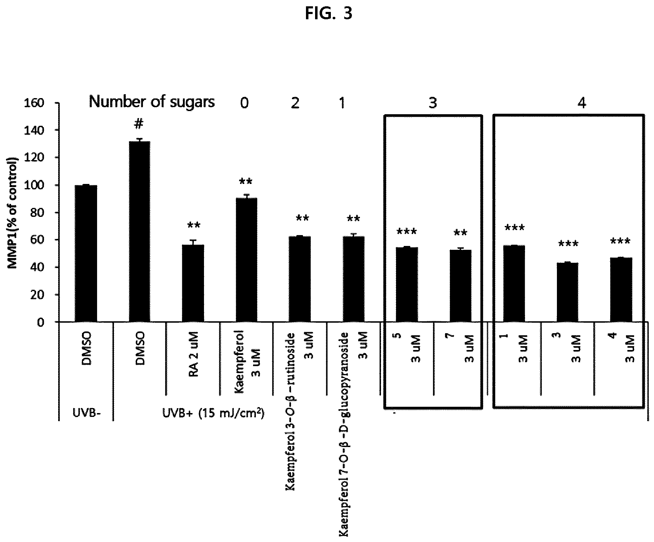

HDF cells were seeded onto a 24-well plate at a concentration of 2.5x10⁴ cells/well and were culture for 24 hours under a cell culturing condition. Then, the cells were cultured for 48 hours after treating with a test substance diluted in FBS-free DMEM. After washing with Dulbecco's phosphate-buffered saline (DPBS) and irradiating UVB (15 mJ/cm²), the medium was replaced with the test substance (2 µM or 3 µM) diluted in DMEM and the cells were cultured for 48 hours. Then, the supernatant was recovered and the amount of MMP-1 was measured using a human total MMP-1 ELISA kit (R&D Systems). The result is shown in FIG. 3.

Referring to FIG. 3, it can be seen that the kaempferol tetrasaccharide compounds (Chemical Formulas 1, 3 and 4) of the present disclosure have superior effect of inhibiting MMP-1 expression as compared to the kaempferol trisaccharide compounds (Chemical Formulas 5 and 7) or retinoic acid (RA) groups.

### Test Example 4: Improvement of skin barrier

### 1) 3D organotypic skin epidermis

Human epidermis models (EpiDerm^{™} EPI-201; MatTek Corporation, Ashland, MA, USA) derived from young (21 years old, denoted by 21Y) and old (58 years old, denoted by 58Y) donors were purchased. The human epidermis model derived from the old donor was treated with the kaempferol tetrasaccharide represented by Chemical Formula 1 at 3 µM for 4 days. Paraffin blocks were prepared after fixing with 10% neutral buffered formalin.

### 2) Investigation of skin barrier markers

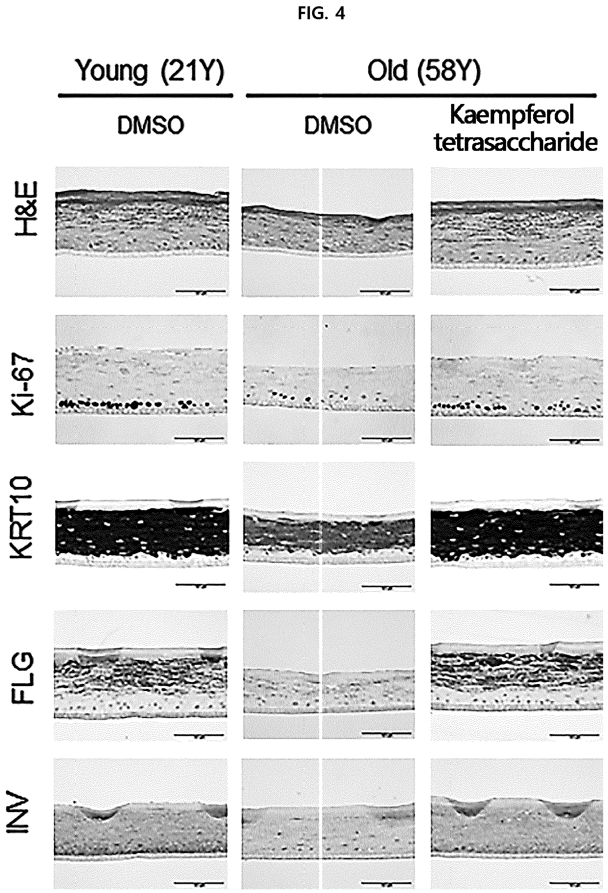

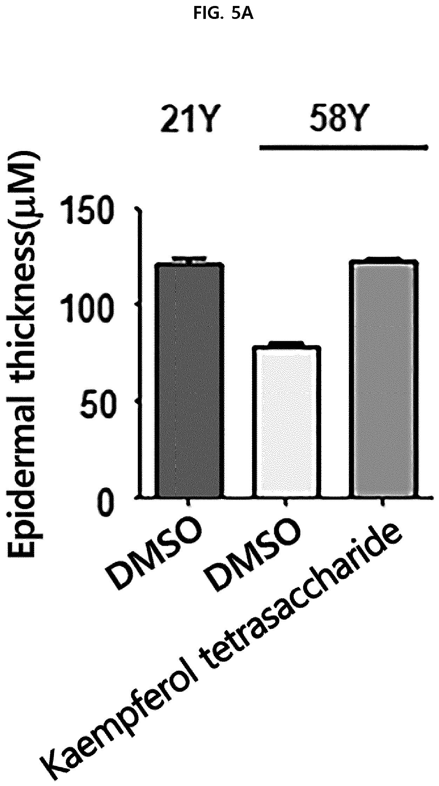

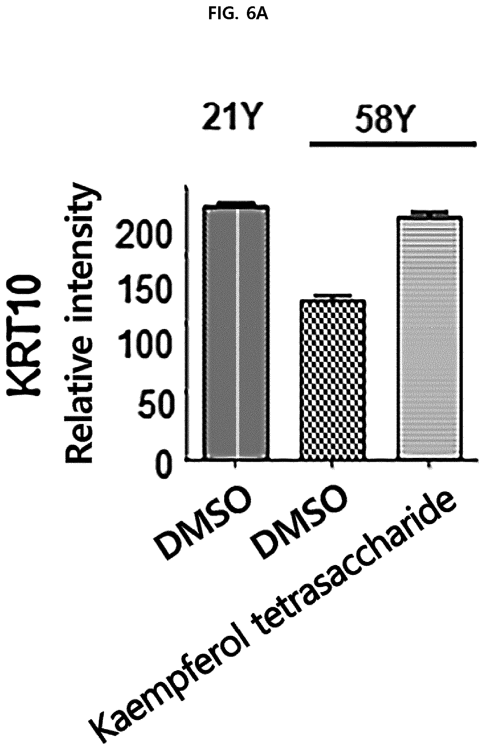

After reacting with anti-KRT 10 (Biolegend, San Diego, CA), anti-FLG (Novus Biologicals, Centennial CO, USA), anti-Involucrin and anti-Ki67 (Abcam) antibodies, the expression of the antibodies was analyzed using a microscope. Specifically, the epidermal tissue derived from the young donor (Young, 21Y) and the epidermal tissue derived from the old donor (Old, 58Y) were treated with the test substance and then stained with the antibodies described above. The result is shown in FIG. 4, FIGS. 5A-5B and FIGS. 6A-6C. FIGS. 5A-5B and FIGS. 6A-6C show the result of quantifying the result of FIG. 4.

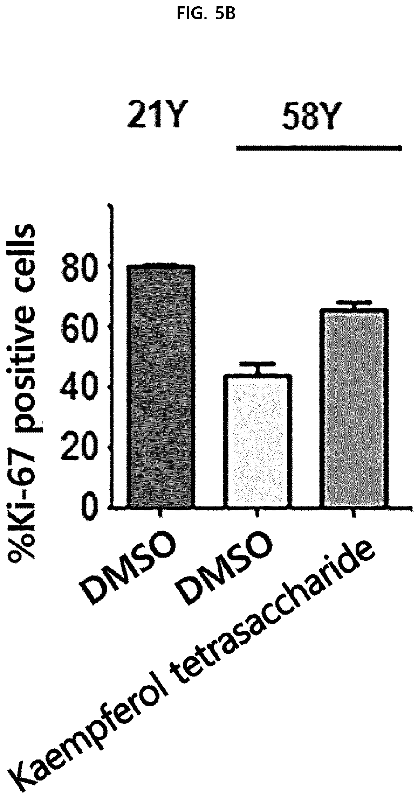

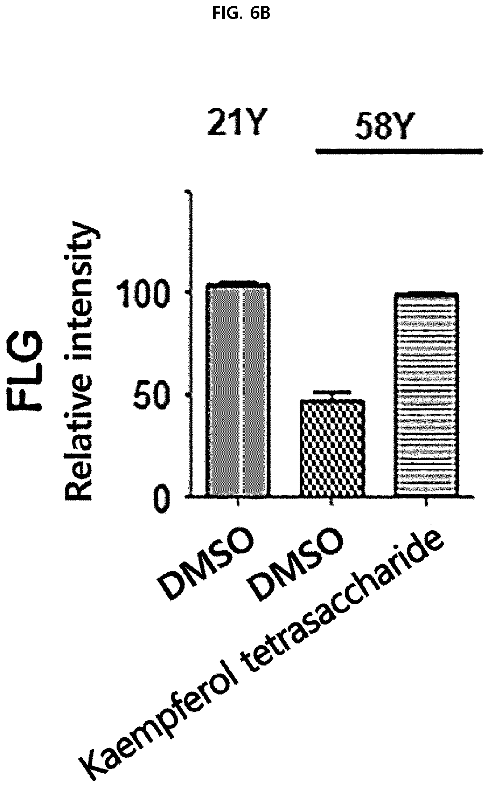

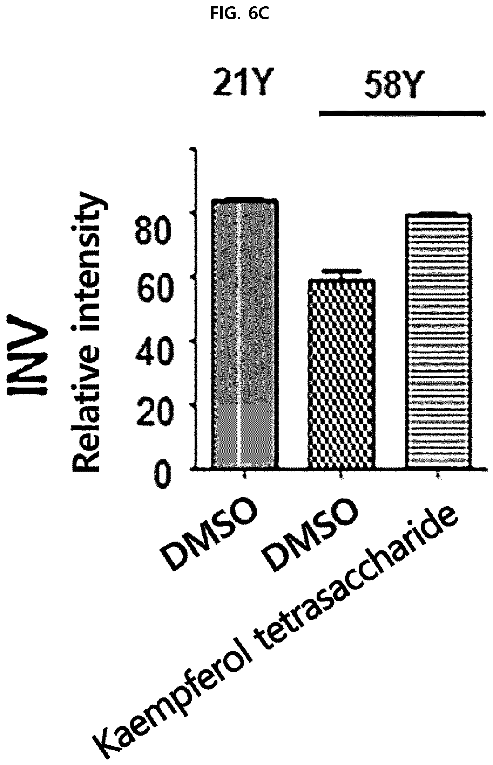

Referring to the figures, it can be seen that the old epidermal tissue had decreased epidermal thickness (FIG. 5A), weakened barrier function (KRT10-FIG. 6A, FLG-FIG. 6B, INV-FIG. 6C) and decreased expression of the regeneration marker (KI67-FIG. 5B) as compared to the young epidermal tissue. However, when the old epidermal tissue was treated with the compound represented by Chemical Formula 1, all of the epidermal thickness, barrier function and expression of the regeneration marker were recovered to levels comparable to those of the young epidermal tissue.

### Test Example 5: Recovery of regeneration ability upon PDK1 inhibition

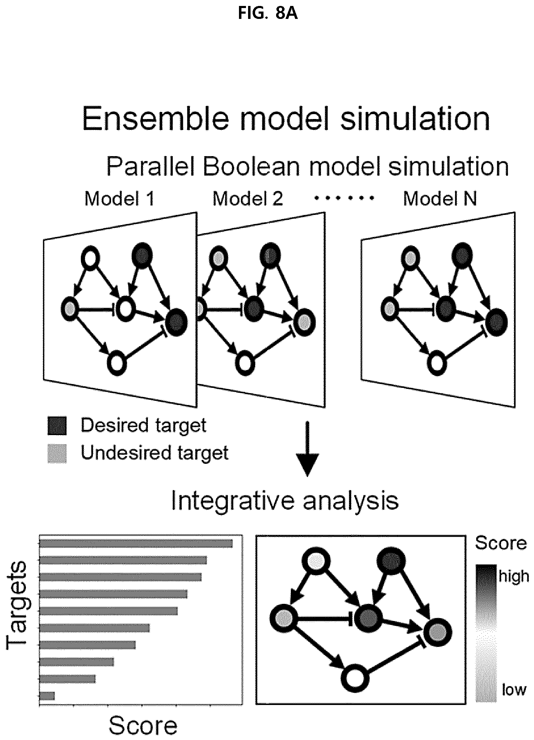

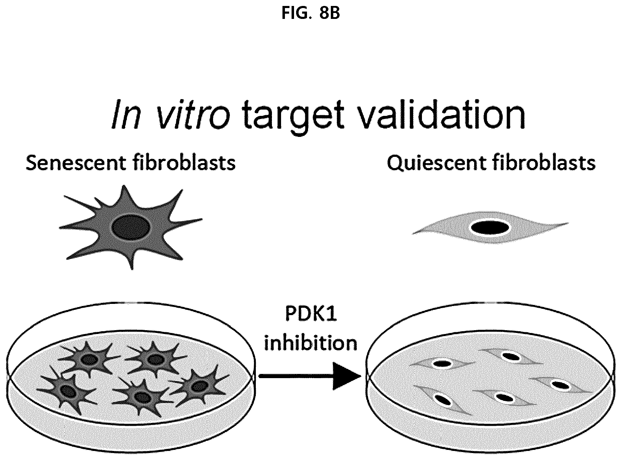

From the analysis of the regulatory signaling network, PDK1 was predicted to be an inhibitor target that can convert senescent fibroblasts to quiescent fibroblasts (FIG. 8A). To validate this prediction, experiment was conducted with normal human dermal fibroblasts (NHDF) exposed to PDK1 inhibitors (FIG. 8B), which eliminated the characteristics of cellular senescence, restored the proliferation of the cells in response to growth factors, and restored skin regeneration ability in the 2D culture and the 3D skin equivalent model.

Collectively, it was confirmed that PDK1 inhibition reduces both cell growth and SASP, which are key processes in maintaining the senescence phenotype, and deactivates the key positive feedback loop composed of PDK1, AKT, IKBKB and PTEN, which are associated with cellular growth. The resulting change in signal transduction restores the quiescence phenotype.

In the following experiments, normal human dermal fibroblasts (NHDF; Lonza, Switzerland) were cultured in Dulbecco's modified Eagle's medium (DMEM, GIBCO 1210-0038) containing 10% fetal bovine serum, in a 5% CO₂ incubator at 37 °C. Cells at passages 6-9 were denoted as young cells, and they were subcultured by 1:5 subculturing until passage 37 to obtain senescent dermal fibroblasts.

### 1) Functional recovery of skin cells due to PDK1 inhibition analyzed by wound healing assay

First, in order to determine if true regeneration ability, not just a reduction in the characteristics of senescence, was achieved by PDK1 inhibition or dual mTOR and NF-κB inhibition, the capacity of the reverted cells was examined for 2D wound healing and 3D skin regeneration.

A detailed experimental procedure is as follows.

Young NHDFs were not subjected to senescence condition or drug treatment. The young NHDFs were analyzed when confluence reached about 80% at PDL 12. Other NHDFs were induced into senescence by treating with doxorubicin (100 ng/mL) and IGF-1 (100 ng/mL) for 7 days. For drug treatment condition, the senescent cells were then exposed to DMSO, rapamycin (100 nM), rapamycin and JSH (2.5 µM) or BX795 (100 nM) for 7 days. For conditioned medium treatment (BX795 + CM), the cells were grown in the presence of doxorubicin and IGF-1 for 7 days, followed by 7 days in the additional presence of doxorubicin, IGF-1 and BX795, were subcultured, and then were incubated with a conditioned medium extracted from the senescent cells for 3 more days.

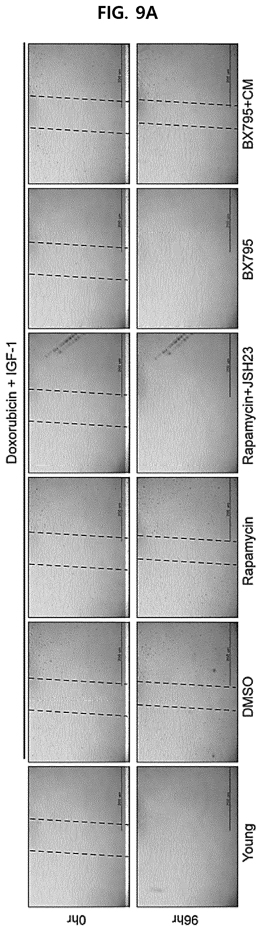

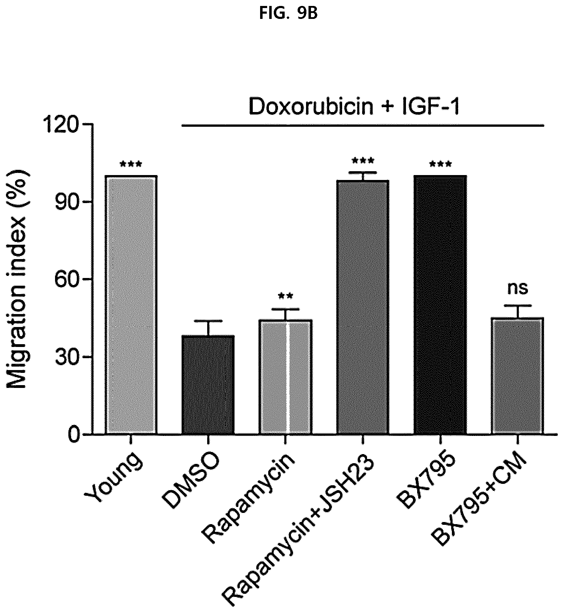

The result is shown in FIGS. 9A-9B. FIG. 9A shows the representative images from 0 hour and 96 hours after wounding. Dashed lines mark the wound area. Scale bar = 200 µm. FIG. 9B shows the result of quantification analysis of migration area in scratch wound assay. Mean ± SD (n = 3), *** P < 0.001 compared to DMSO control senescent cells by one-way analysis of variance.

PDK1 inhibition or dual inhibition of mTOR and NF-κB (but not single inhibition of mTOR) facilitated the treatment of the scratch wound of NHDF monolayers (FIGS. 9A-9B).

### 2) Effect of PDK1 on skin regeneration

3D skin models (skin equivalents) were prepared from young NHDFs (PDL 12) or senescent NHDFs induced into senescence by doxorubicin and IGF-1 or repeated plating (replicative senescence, PDL 57) and were exposed to DMSO or BX795. The treatment concentrations are as follows: doxorubicin (100 mg/mL), IGF-1 (100 ng/mL), BX-795 (100 nM).

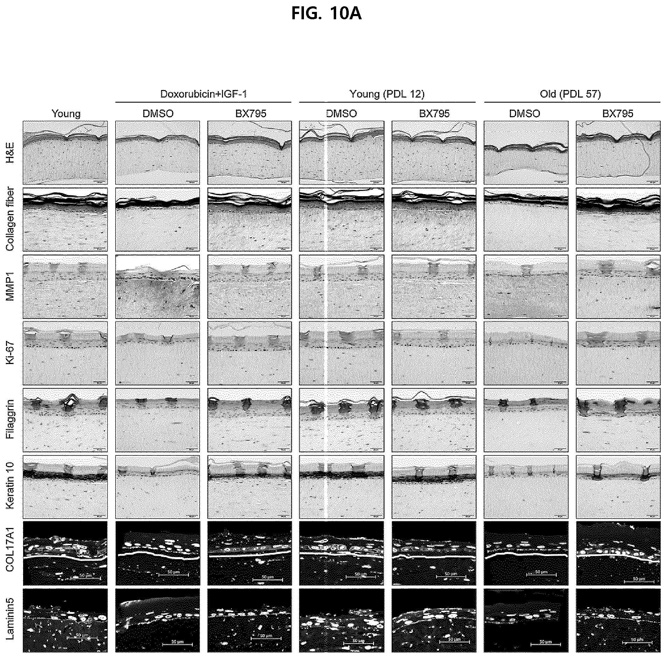

FIG. 10A shows the skin equivalent model sections stained with hematoxylin and eosin (H&E) or Masson's trichrome to detect collagen fibers. The indicated proteins were detected by immunohistochemistry. The results are from three representative experiments. Scale bar = 50 µm.

Specifically, the experiment was conducted as follows.

Paraffin samples were dewaxed in xylene and rehydrated through a descending ethanol to water series (3 minutes per solution). Epitope retrieval was performed at 121 °C for 10 minutes using a citrate buffer (pH 6.0, antigen retriever, 64142; Electron Microscopy Sciences, Hatfield, PA) and the slides were cooled prior to washing 3 times in PBS/0.05% Tween 20. The sections were then incubated with water/1% H₂O₂ for 30 minutes to block endogenous peroxidase, rinsed with water and PBS/0.05% Tween 20 for 5 minutes, and incubated with PBS/10% goat serum (Dako, Glostrup, Denmark) for 20 minutes before exposure to the following antibodies overnight at 4 °C; Primary antibodies are: anti-KRT 10 (Biolegend, San Diego, CA), anti-FLG (Abcam), anti-MMP-1(Abcam) and anti-Ki67 (Abcam).

The samples were washed thoroughly with water for 10 minutes and then with PBS/0.05% Tween 20 for 5 minutes, and incubated with EnVision Systems horse radish peroxidase-labeled polymer anti-mouse antibody (K4001; Dako, Carpinteria, CA) for 30 minutes. Then, the samples were washed with water and PBS for 3-5 minutes before incubation with DAB+ Substrate Chromogen System (K3468; Dako). The reaction was stopped by rinsing with tap water, and the sections were counterstained with Mayer's hematoxylin for 3-5 minutes and rinsed with tap water for 1 minute and then with Scott's bluing water for 2 minutes.

Finally, the sections were dehydrated in graded ethanol, cleared in xylene and mounted in Cytoseal 60 (Thermo Scientific, Waltham, MA). Masson's trichrome staining was performed using a commercially available kit (BBC Biochemical, Seattle, WA, USA). For immunofluorescence staining, antigen retrieval was performed by boiling the slides for 20 minutes in Dako target retrieval solution (Dako). Nonspecific staining was blocked by incubation with PBS containing 10% donkey serum and 0.05% Triton X-100 for 30 minutes. Tissue sections were incubated with COL17A1 (Abcam) and laminin-5 (Santa Cruz Biotechnology) in Dako fluorescence mounting medium (Dako) at 4 °C overnight. Subsequently, the sections were incubated with appropriate secondary antibodies conjugated with Alexa Fluor 488 (Invitrogen-Molecular Probes) for 2 hours at room temperature. After washing with PBS, 4',6-diamidine-2'-phenylindole dihydrochloride (DAPI, Invitrogen-Molecular Probes) was added for nuclear counterstaining. Coverslips were mounted on glass slides using a fluorescent mounting medium (Thermo Electron).

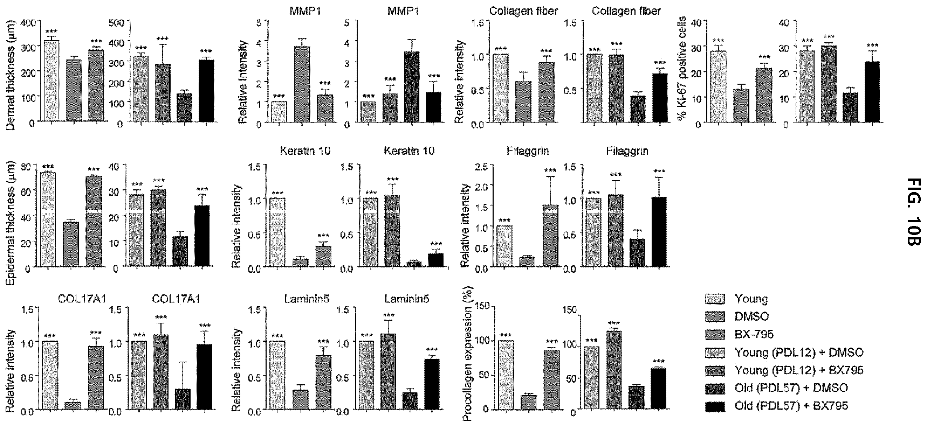

FIG. 10B shows, sequentially from top left, the result of quantifying dermal thickness using the ImageJ software, quantifying MMP1, quantifying pan-collagen fiber detected by Masson's trichrome staining, quantifying Ki-67-positive cells, quantifying epidermal thickness using the ImageJ software, quantifying keratin 10, quantifying filaggrin, quantifying COL17A1, quantifying laminin-5, and quantifying the type I procollagen protein secreted into the culture medium by ELISA. The data in FIG. 10B are shown as mean ± SD of three independent fields obtained from three independent samples. ***P <0.001 compared to DMSO control senescent cells by one-way analysis of variance.

PDK1 inhibition reversed the aging of the dermis associated with both replicative senescence and chemically induced senescence, as evidenced by the attenuation of the increase in MMP1, the decrease in collagen fibers, and the reduction in secretion of procollagen (FIGS. 10A-10B). For epidermal homeostasis, the balance between keratinocyte proliferation and differentiation is regulated by dermal fibroblasts.

Therefore, it was investigated whether the reversion of senescent dermal fibroblasts by PDK1 inhibition affects the keratinocyte proliferation and differentiation. BX-795 is a potent PDK1 inhibitor that blocks PDK1/Akt signaling in tumor cells and inhibits the anchorage-dependent growth of a variety of tumor cell lines during culture or induced apoptosis. The proliferation marker Ki-67 was markedly increased in the epidermis of the skin equivalents treated with BX795, which is known as a PDK1 inhibitor, indicating the increased proliferative capacity of keratinocytes, which preserved epidermal thickness in the skin equivalents from the senescence-induced NDHFs (FIGS. 10A-10B).

In addition, the intensity of staining was compared for the keratinocyte differentiation markers (keratin 10 and filaggrin) and the markers for basement membrane in the dermal-epidermal junction (COL17A1 and laminin 5). As a result, PDK1 inhibition recovered the reduced abundance of these keratinocyte differentiation markers and the components of the dermal-epidermal junction found in the skin equivalents from the senescent NHDFs (FIG. 10A).

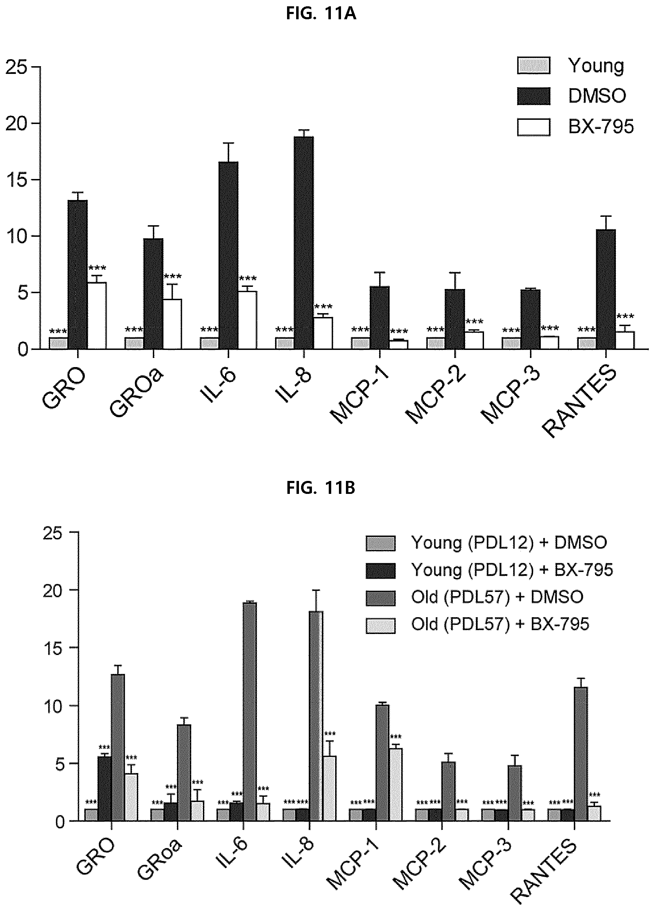

Similarly to the observations with the 2D senescent NHDFs (FIGS. 9A-9B), the conditioned medium from the skin equivalents prepared from either type of senescent NHDFs treated with BX795 contained less GRO, GROα, IL-6, IL-8, MCP-1 to 3 and RANTES than was present in the conditioned medium from the vehicle-treated skin equivalents (FIGS. 11A-11B).

Specifically, experiment was conducted as follows.

PDK1 inhibition inhibits SASP in skin equivalents. 3D skin models (skin equivalents) were prepared from young NHDFs (PDL 12) or senescent NHDFs induced into senescence by doxorubicin and IGF-1 or repeated plating (replicative senescence, PDL 57), and were exposed to DMSO or BX795. The treatment concentrations are as follows: doxorubicin (100 mg/mL), IGF-1 (100 ng/mL), BX-795 (100 nM).

The conditioned media (CM) of skin equivalents from chemically induced or replicative senescent NHDFs with or without BX795 treatment were measured with a cytokine antibody array and were normalized to internal positive controls. FIG. 11A shows secreted proteins measured in a cytokine array of the skin equivalents from the chemically induced NHDFs treated with or without BX795. FIG. 11B shows secreted proteins measured in a cytokine array of the skin equivalents from the replicative senescent NHDFs treated with or without BX795. Mean ± SD (n = 3), ***P < 0.001 compared to DMSO control senescent cells by one-way analysis of variance.

Altogether, these findings indicate that PDK1 inhibition converts senescent dermal fibroblasts to quiescent fibroblasts, which enhances renewal of the epidermis and preserves skin thickness to limit skin deterioration associated with aging.

## Claims

1. A composition comprising a kaempferol saccharide compound represented by any of Chemical Formulas 1 to 4, an isomer thereof, a pharmaceutically acceptable salt thereof, a hydrate thereof or a solvate thereof as an active ingredient: 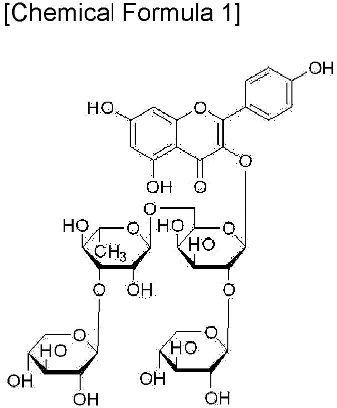 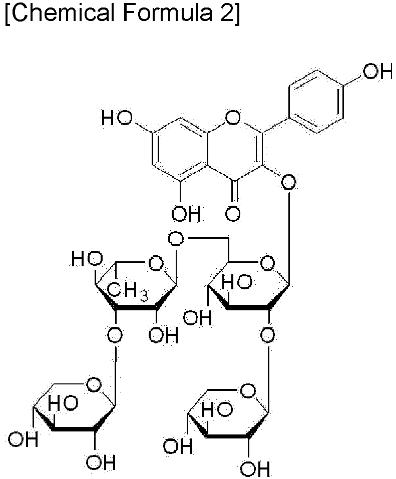 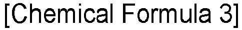 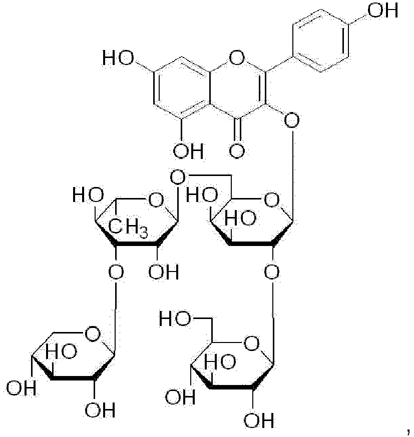 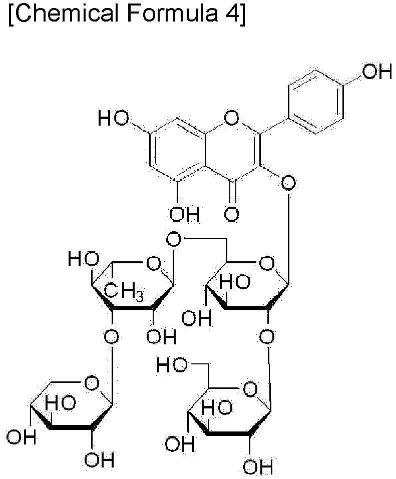 for use in skin protection, wherein the skin protection is improving, preventing or treating atopic skin.

2. The composition for use in skin protection according to claim 1, wherein the kaempferol saccharide compound is extracted from *Camellia japonica* seed cake.

3. The composition for use in skin protection according to claims 1 or 2, wherein the concentration of the active ingredient is 0.01-100 µM based on the total weight of the composition.

4. The composition for use in skin protection according to any of claims 1-3, wherein the composition is a pharmaceutical composition for improving, preventing or treating atopic dermatitis.

5. The composition for use in skin protection according to claim 4, wherein the administration dosage of the active ingredient is 0.01-100 mg/kg/day.

6. A non-therapeutic use of a composition comprising a kaempferol saccharide compound represented by any of Chemical Formulas 1 to 4, an isomer thereof, a pharmaceutically acceptable salt thereof, a hydrate thereof or a solvate thereof as an active ingredient: 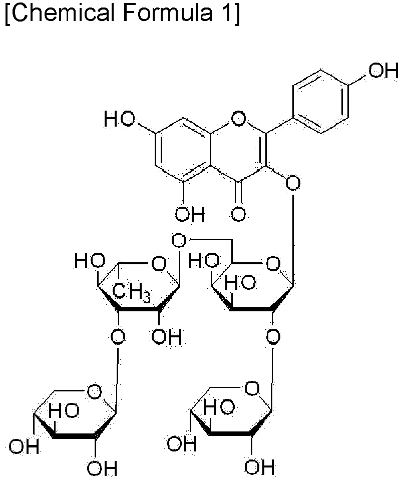 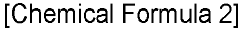 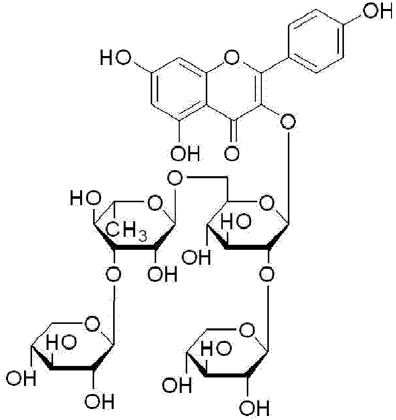 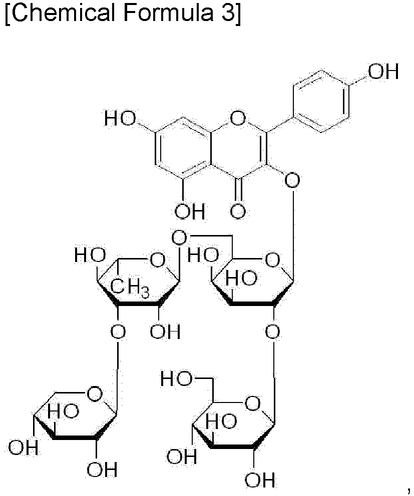 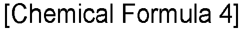 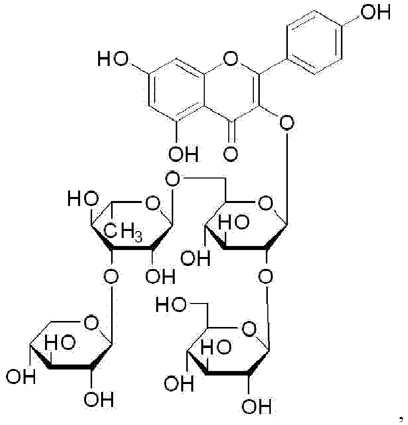 for use in skin protection, wherein the skin protection is reducing skin cornification cycle, improving skin barrier or inducing growth of skin cells whose growth has been inhibited due to aging.

7. The non-therapeutic use according to claim 6, wherein the kaempferol saccharide compound is extracted from *Camellia japonica* seed cake.

8. The non-therapeutic use according to claims 6 or 7, wherein the concentration of the active ingredient is 0.01-100 µM based on the total weight of the composition.

9. The non-therapeutic use according to any of claims 6-8, wherein the composition is a cosmetic composition.

10. The non-therapeutic use according to any of claims 6-8, wherein the composition is a food composition.

## Patentansprüche

1. Zusammensetzung, die eine Kaempferolsaccharidverbindung umfasst, die durch eine der chemischen Formeln 1 bis 4, einem Isomer davon, einem pharmazeutisch akzeptablem Salz davon, einem Hydrat davon oder einem Solvat davon als Wirkstoff repräsentiert ist: 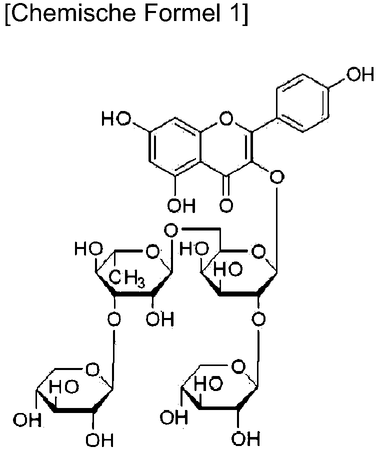 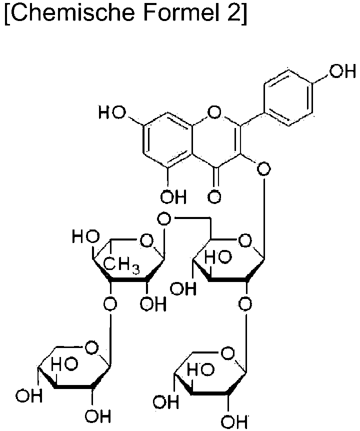 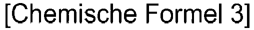 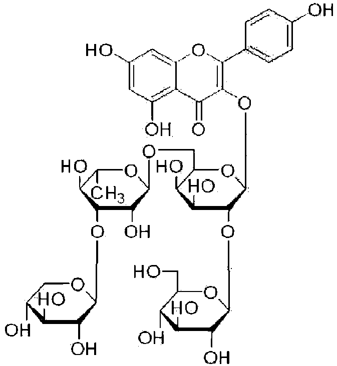 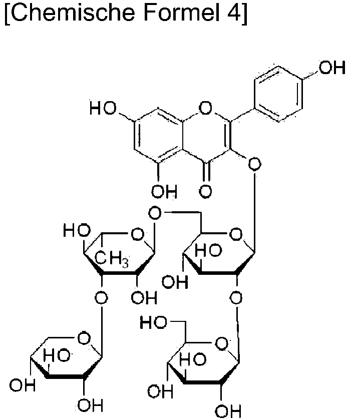 zur Verwendung im Hautschutz, wobei der Hautschutz der Verbesserung, Vorbeugung oder Behandlung atopischer Haut dient.

2. Zusammensetzung zur Verwendung zum Hautschutz nach Anspruch 1, wobei die Kaempferolsaccharidverbindung aus *Camellia-Japonica-Samenkuchen* extrahiert wird.

3. Zusammensetzung zur Verwendung zum Hautschutz nach Anspruch 1 oder 2, wobei die Konzentration des Wirkstoffs 0,01-100 µM basierend auf dem Gesamtgewicht der Zusammensetzung beträgt.

4. Zusammensetzung zur Verwendung im Hautschutz nach einem der Ansprüche 1 bis 3, wobei die Zusammensetzung eine pharmazeutische Zusammensetzung zur Verbesserung, Vorbeugung oder Behandlung atopischer Dermatitis ist.

5. Zusammensetzung zur Verwendung im Hautschutz nach Anspruch 4, wobei die Verabreichungsdosis des Wirkstoffs 0,01-100 mg/kg/Tag beträgt.

6. Nichttherapeutische Verwendung einer Zusammensetzung, die eine Kaempferolsaccharidverbindung umfasst, die durch eine der chemischen Formeln 1 bis 4, einem Isomer davon, einem pharmazeutisch akzeptablem Salz davon, einem Hydrat davon oder einem Solvat davon als Wirkstoff repräsentiert ist: 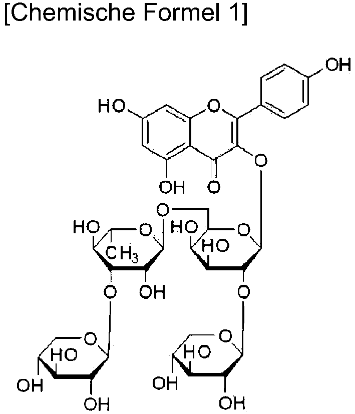 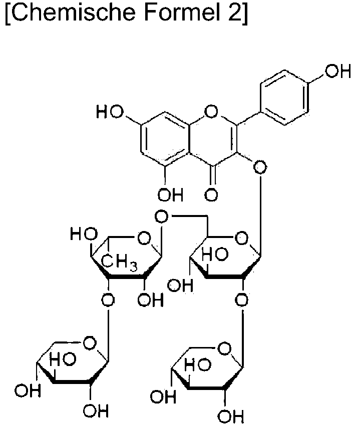 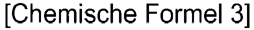 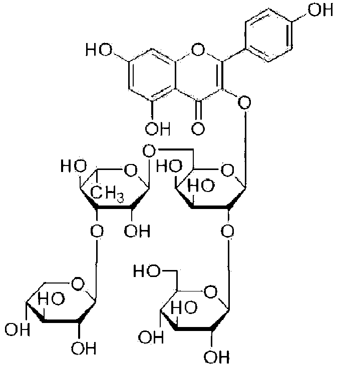 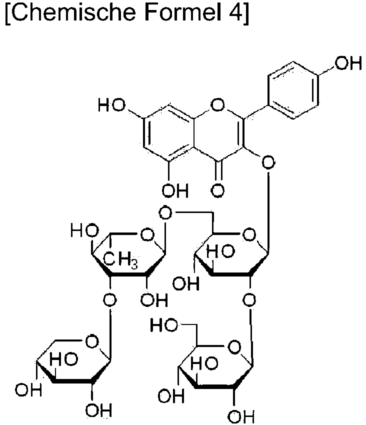 zur Verwendung im Hautschutz, wobei der Hautschutz den Hornbildungszyklus der Haut reduziert, die Hautbarriere verbessert oder das Wachstum von Hautzellen induziert, deren Wachstum durch Alterung gehemmt wurde.

7. Nichttherapeutische Verwendung nach Anspruch 6, wobei die Kaempferolsaccharidverbindung aus *Camellia-Japonica-Samenkuchen* extrahiert wird.

8. Nichttherapeutische Verwendung nach Anspruch 6 oder 7, wobei die Konzentration des Wirkstoffs 0,01-100 µM basierend auf dem Gesamtgewicht der Zusammensetzung beträgt.

9. Nichttherapeutische Verwendung nach einem der Ansprüche 6 bis 8, wobei die Zusammensetzung eine kosmetische Zusammensetzung ist.

10. Nichttherapeutische Verwendung nach einem der Ansprüche 6 bis 8, wobei die Zusammensetzung eine Lebensmittelzusammensetzung ist.

## Revendications

1. Composition comprenant un composé saccharidique de kaempférol représenté par l'une des Formules Chimiques 1 à 4, un isomère de celui-ci, un sel pharmaceutiquement acceptable de celui-ci, un hydrate de celui-ci ou un solvate de celui-ci comme principe actif : 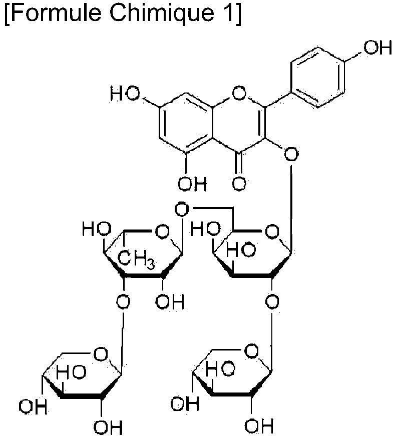 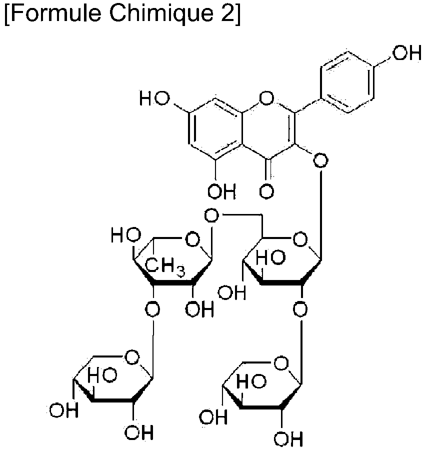 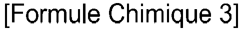 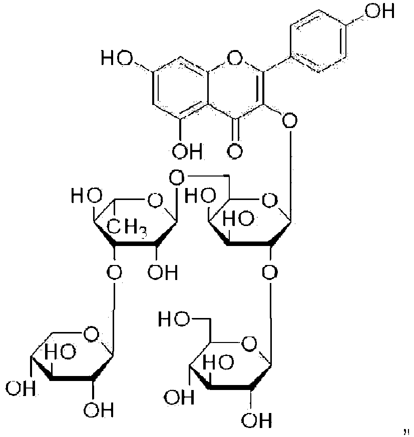 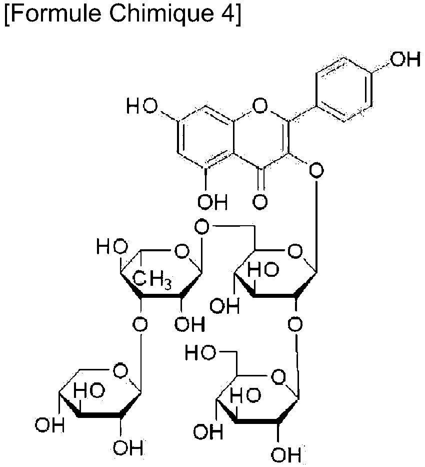 pour une utilisation dans la protection de la peau, dans laquelle la protection de la peau consiste à améliorer, prévenir ou traiter la peau atopique.

2. Composition pour une utilisation dans la protection de la peau selon la revendication 1, dans laquelle le composé saccharidique de kaempférol est extrait du tourteau de graines de *Camellia japonica.*

3. Composition pour une utilisation dans la protection de la peau selon la revendication 1 ou 2, dans laquelle la concentration du principe actif est de 0,01 à 100 µM par rapport au poids total de la composition.

4. Composition pour une utilisation dans la protection de la peau selon l'une des revendications 1 à 3, dans laquelle la composition est une composition pharmaceutique destinée à améliorer, prévenir ou traiter la dermatite atopique.

5. Composition pour une utilisation dans la protection de la peau selon la revendication 4, dans laquelle la posologie d'administration du principe actif est de 0,01 à 100 mg/kg/jour.

6. Utilisation non thérapeutique d'une composition comprenant un composé saccharidique de kaempférol représenté par l'une des Formules Chimiques 1 à 4, un isomère de celui-ci, un sel pharmaceutiquement acceptable de celui-ci, un hydrate de celui-ci ou un solvate de celui-ci comme principe actif : 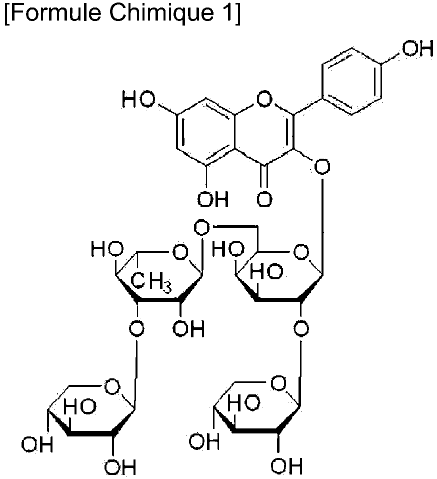 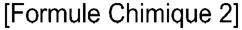 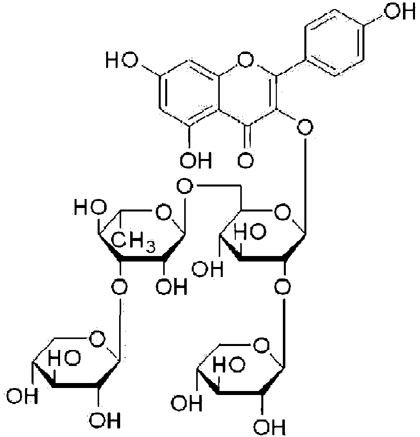 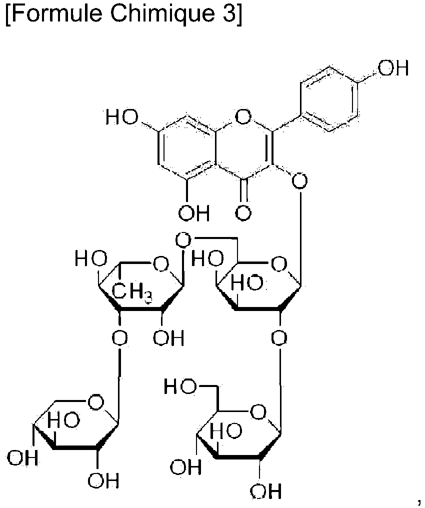 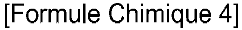 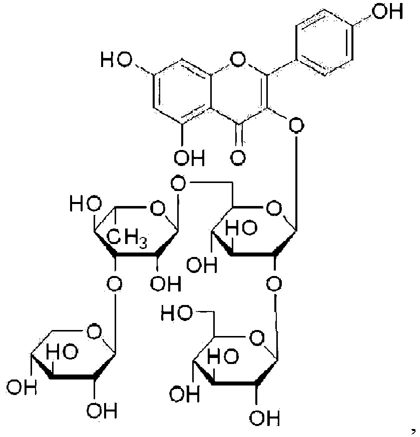 pour une utilisation dans la protection de la peau, dans laquelle la protection de la peau consiste à réduire le cycle de kératinisation cutanée, améliorer la barrière cutanée ou induire la croissance de cellules cutanées dont la croissance a été inhibée en raison du vieillissement.

7. Utilisation non thérapeutique selon la revendication 6, dans laquelle le composé saccharidique de kaempférol est extrait du tourteau de graines de *Camellia japonica.*

8. Utilisation non thérapeutique selon la revendication 6 ou 7, dans laquelle la concentration du principe actif est de 0,01 à 100 µM par rapport au poids total de la composition.

9. Utilisation non thérapeutique selon l'une des revendications 6 à 8, dans laquelle la composition est une composition cosmétique.

10. Utilisation non thérapeutique selon l'une des revendications 6 à 8, dans laquelle la composition est une composition alimentaire.
